(19) Europäisches Patentamt
European Patent Office
Office européen des brevets

(11) **EP 4 431 525 A1**

(12) **EUROPEAN PATENT APPLICATION**

(43) Date of publication:
**18.09.2024 Bulletin 2024/38**

(21) Application number: **23162288.7**

(22) Date of filing: **16.03.2023**

(51) International Patent Classification (IPC):
**C07K 16/24** (2006.01)  **C07K 16/28** (2006.01)

(52) Cooperative Patent Classification (CPC):
**C07K 16/246; C07K 16/2818;** A61K 2039/505;
C07K 2317/24; C07K 2317/31; C07K 2317/33;
C07K 2317/41; C07K 2317/55; C07K 2317/622;
C07K 2317/73; C07K 2317/90; C07K 2317/92;
C07K 2319/00

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Anaveon AG**
**4057 Basel (CH)**

(72) Inventors:
• **HUBER, Christoph**
  **4057 Basel (CH)**
• **KATOPODIS, Andreas**
  **4057 Basel (CH)**
• **MURER, Patrizia**
  **4057 Basel (CH)**

(74) Representative: **Schulz Junghans**
**Patentanwälte PartGmbB**
**Großbeerenstraße 71**
**10963 Berlin (DE)**

Remarks:
The complete document including Reference
Table(s) and the Sequence Listing(s) can be
downloaded from the EPO website

(54) **IL-2 FUSION PROTEIN**

(57) The invention relates to PD-1:PD-L1 non-blocking immunoconjugates comprising an IL-2 polypeptide providing a biased signal to the dimeric IL-2 receptor, and a PD-1 targeting domain, and their use for treating cancer.

**Description**

Field

The present invention relates to an IL-2 fusion protein designed to target exhausted T cells expressing PD-1. The invention further relates to pharmaceutical compositions comprising the IL-2 fusion protein, and methods of treating cancer patients with same, optionally in combination with PD-1 or PD-L1 antagonist antibodies.

Background

Recombinant IL-2 (aldesleukin) activates IL-2 receptors expressed on effector T cell subsets including CD8+ T cells, and NK cells. In its native form IL-2 is a 15 kDa protein that can signal through the high affinity trimeric receptor IL-2R alpha/beta/gamma [composed of the IL-2R alpha chain (CD25), the IL-2R beta chain (CD122) and the IL-2R gamma chain (CD132)] or through the medium affinity dimeric receptor IL-2R beta/gamma. Regulatory T cells (Tregs) and lung endothelial cells express CD25 and predominantly utilize the high affinity trimeric receptor while CD8+ T cells and NK cells (effector cells) do not express CD25 under steady state and display the dimeric receptor on their cell surface. This receptor expression profile limits the potential antitumor responses generated by treatment with aldesleukin, as immunosuppressive Tregs rather than CD8+ T cells and NK cells are preferentially stimulated, and lung endothelial cell activation through IL-2R contributes to the strong, IL-2 associated, side-effects such as hypotension and vascular leak syndrome.

One way to improve the efficacy of aldesleukin has been to develop molecules with biased signaling towards the medium affinity IL-2 receptors expressed by effector cells and limited activation of the high affinity IL-2 receptor expressed by Tregs which may suppress anti-tumor immunity. The anti-tumor activity of such receptor-biased IL-2 molecules can be further improved by targeting the molecule to the tumor or to the tumor microenvironment, for example, by fusing the cytokine to antibodies binding tumor antigens. These molecules present IL-2 across cells and are often called trans-signaling IL-2 bispecifics. IL-2 constructs targeted to tumor tissues were shown to have a synergistic efficacy when used to treat cancer models in combination with a checkpoint inhibition antibody targeting PD-L1 (Klein C. 2017 Oncoimmunology 6:e1277306). Alternatively, receptor-biased IL-2 molecules can be targeted to CD8 T cells directly by using targeting antibodies binding to CD8, PD-1 or other antigens expressed on the surface of CD8 T cells. Because these constructs present IL-2 from the same T cell surface, they are often called cis-signaling IL-2 bispecifics. Such *cis*-signaling fusion proteins have been generated using anti-PD-1/IL-2 bispecifics, (WO2018184964A1, Deak L. C. et al. 2022, Nature 610:161) and were shown to have superior efficacy in mouse pre-clinical models. In all the known anti-PD-1/IL-2 constructs, the targeting arm consists of blocking anti-PD-1 antibodies (blocking PD-1/PD-1L interactions). Such a targeting arm has the potential advantage of relieving PD-L1 induced T cell suppression and delivering a proliferative IL-2 signal to the same effector cells. There is, however, an inherent difficulty of pharmacologically administering a blocking therapeutic (requiring full coverage of the target) and an agonistic therapeutic (requiring agonistic dosing and frequency). In the specific case of PD-1 and IL-2 therapeutics, anti-PD-1 antibodies are administered in mg/kg doses to ensure full target coverage, but IL-2 principles are administered in microgram/kg doses to avoid overstimulation and on-target toxicity. The discrepancy may be addressed by mutating the IL-2 portion of the molecule to reduce affinity to its receptor and thereby allow higher doses of the bispecific. Mutations to the IL-2 structure, however, may result in anti-drug antibodies which can render the therapy ineffective or toxic. Furthermore, in patients receiving PD-1 check point inhibitor (CPi) therapy, the epitope targeted by the PD-1/IL-2 fusion protein is already occupied by the CPi thus preventing binding of the fusion protein to the targeted cells.

Based on the above-mentioned state of the art, the objective of the present invention is to provide an IL-2 molecule with improved therapeutic characteristics. This objective is attained by the subject-matter of the independent claims of the present specification, with further advantageous embodiments described in the dependent claims, examples, figures and general description of this specification.

Summary of the Invention

The inventors sought to develop a PD-1 targeted therapeutic IL-2 molecule which does not inhibit the binding or antagonist action of conventional PD-1, or PD-L1 targeted checkpoint inhibitor antibodies. The inventors have generated non-blocking anti-PD-1 antibodies for targeting IL-2 to PD-1 expressing cells. The anti-PD-1 binding domains of non-blocking antibodies are used as a targeting moiety in an immunoconjugate further comprising an IL-2 polypeptide, delivering IL-2 signal to the appropriate exhausted CD8 T cells, and allowing optimal combination with existing anti-PD1 therapeutics. The resulting compound provides highly targeted IL-2 signaling to exhausted T cells, and may be combined more flexibly with existing CPi treatment regimens targeting the PD-1/PD-L1 signaling axis. This results in an immunoconjugate compound that effectively delivers IL-2 stimulation, while allowing modulated dosing of potent active agents to achieve safe and efficacious treatment of cancer.

One aspect of the invention is an immunoconjugate comprising an anti-PD-1 binding domain, and an anti-IL-2 antigen binding domain. Said anti-PD-1 binding domain comprises a heavy chain polypeptide and a light chain polypeptide derived from an anti-PD-1 antibody (an antibody capable of binding specifically to the extracellular domain of the human PD-1 protein). Said anti-IL-2 binding domain comprises a heavy chain polypeptide and a light chain polypeptide derived from an anti-IL-2 antibody (an antibody capable of binding specifically to the human IL-2 protein). One of the antibody variable domains within the heavy chain, or the light chain of the anti-IL-2 antigen binding domain is joined, optionally by means of one or two peptide linkers, to an IL-2 polypeptide.

In particular embodiments, the IL-2 is human IL-2, an artificial IL-2 variant polypeptide such as Proleukin, or an IL-2 mutein polypeptide. In certain embodiments, the IL-2 polypeptide is a circularly permuted IL-2 polypeptide (IL2CP), characterized by rearranged amino acid residues relative to the human IL-2 sequence. Together, the anti-IL-2 binding domain joined to the IL-2 polypeptide delivers biased IL-2 signaling to the dimeric IL-2 receptor expressed on target cells.

In particular embodiments, the anti-PD-1 binding domain incorporated into the immunoconjugate according to the invention is derived from a non-blocking PD-1 antibody. In other words, binding of the immunoconjugate to a cell expressing PD-1 does not significantly inhibit binding of PD-1 to its natural ligand PD-L1, or PD-1 antagonist compounds, such as the antibodies nivolumab, or pembrolizumab. This feature allows the immunoconjugate according to the invention, to be used as a medicament in combination with CPi compounds which inhibit checkpoint blockade by binding to either PD-1, or PD-L1.

In particular embodiments of the immunoconjugate according to the invention, the IL-2 polypeptide is an IL2CP embedded within the variable domain of the heavy or light chain of the anti-IL-2 binding domain, to provide a single fusion polypeptide. In alternative embodiments, the IL-2 polypeptide is joined to the N' terminal region of the variable domain of either the heavy, or light chain of the anti-IL-2 binding domain by means of a peptide linker, to provide a single fusion polypeptide.

In certain embodiments of the immunoconjugate according to the invention, it comprises an Fc portion, particularly an IgG isotype Fc portion. In particular embodiments, the Fc portion is characterized by amino acid modifications to enhance correct heavy chain pairing, and/or to optimize levels of Fc effector functions.

In further particular embodiments, the immunoconjugate according to the invention is a heterotetrameric IgG (for example, a Crossmab, or kappa/lambda format) wherein one antibody arm comprises the anti-PD-1 binding domain, and one antibody arm comprises the anti-IL-2 antibody binding domain joined to an IL-2 polypeptide.

## Terms and definitions

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

The term *PD-1* in the context of the present specification relates to the human PD-1 protein, encoded by the *PDCD1* gene, also sometimes referred to as CD279 (Uniprot Q15116).

The term *IL2CP, or IL2-CP* in the context of the present specification relates to a circularly permuted IL-2 polypeptide is created by "opening" the IL-2 polypeptide sequence, to create a new N' terminus and C' terminus, and fusing natural N' and C' terminal amino acid residues. This generates a reordered IL2CP polypeptide retaining important tertiary structures of the cytokine that allow signaling through dedicated receptors.

The term *dimeric IL-2 receptor* in the context of the present specification relates to the heterodimer receptor comprising the two receptor chains CD122 and CD132.

Any patent document cited herein shall be deemed incorporated by reference herein in its entirety.

## Sequences

In the context of the present specification, the terms *sequence identity* and *percentage of sequence identity* refer to a single quantitative parameter representing the result of a sequence comparison determined by comparing two aligned sequences position by position. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA.

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment.

*Binding; Binders Ligands Antibodies:*

The term *specific binding* in the context of the present invention refers to a property of ligands that bind to their target with a certain affinity and target specificity. The affinity of such a ligand is indicated by the dissociation constant of the ligand. A specifically reactive ligand has a dissociation constant of $\leq 10^{-7}$mol/L (particularly $\leq 10^{-9}$mol/L) when binding to its target, but a dissociation constant at least three orders of magnitude higher in its interaction with a molecule having a globally similar chemical composition as the target, but a different three-dimensional structure.

In the context of the present specification, the term *dissociation constant ($K_D$)* is used in its meaning known in the art of chemistry and physics; it refers to an equilibrium constant that measures the propensity of a complex composed of [in most cases, two] different components to dissociate reversibly into its constituent components. The complex can be e.g. an antibody-antigen complex AbAg composed of antibody Ab and antigen Ag. $K_D$ is expressed in molar concentration [mol/l] and corresponds to the concentration of [Ab] at which half of the binding sites of [Ag] are occupied, in other words, the concentration of unbound [Ab] equals the concentration of the [AbAg] complex. The dissociation constant can be calculated according to the following formula:

$$K_D = \frac{[Ab] * [Ag]}{[AbAg]}$$

*[Ab]: concentration of antibody, [Ag]: concentration of antigen; [AbAg]: concentration of antibody-antigen complex*

Detailed Description of the Invention

A first aspect of the invention is an immunoconjugate comprising an anti-PD-1 antibody binding domain, an anti-IL-2 antibody antigen binding domain, and an IL-2 polypeptide. The IL-2 polypeptide is covalently linked to amino acid residues of the anti-IL-2 binding domain, optionally by means of one, or two peptide linkers, to provide a single contiguous recombinant polypeptide, resulting in an immunoconjugate biased IL-2 signaling to the dimeric IL-2 receptor. The features of each of these functional components are elaborated upon in the sections below.

*Anti-PD1 binding domains*

The anti-PD1 binding domain of the immunoconjugate according to the invention is the antigen-binding domain portion of an anti-PD-1 antibody. In other words, it comprises a heavy chain polypeptide and a light chain polypeptide derived from an antibody capable of binding specifically to the extracellular domain of the human PD-1 protein. PD-1 specificity is conferred by an antibody heavy chain variable domain polypeptide (PD1-VH), and an antibody light chain variable domain polypeptide (PD1-VL) comprising the antibody CDR and surrounding framework residues.

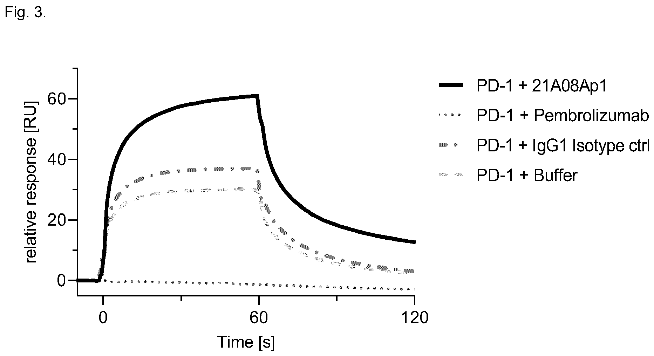

The anti-PD-1 binding domain, and thus the immunoconjugate as a whole, does not block the interaction between PD-1 and current, commercially/clinically used antagonistic CPi antibodies targeting PD-1, including pembrolizumab, and nivolumab. The PD-1 antagonist non-blocking feature of the immunoconjugate, allows the compound to be administered as a therapeutic agent targeted to PD-1+ cells, in combination with existing, anti-PD-1 checkpoint blockade antibodies that are regulatorily approved. The immunoconjugate targets PD-1+ cells to deliver IL-2 signaling to exhausted PD-1+ cells, without disturbing the effects of a co-administered CPi compound (Table 17, Fig. 3). Example 13 further demonstrates that the immunoconjugate does not inhibit the natural ligand for PD-1, PD-L1, from binding to PD-1.

Significant inhibition of a PD-1 antagonist antibody by immunoconjugate binding to said cell can be determined by measuring the % of immunoconjugate binding to a sample of human PD-1+ cells (e.g. Jurkat PD-1+ cells), following preincubation of the sample with 100x molar excess of pembrolizumab, or nivolumab. The mean fluorescence intensity (MFI) of the immunoconjugate in a pembrolizumab, or nivolumab pre-blocked sample, is no more than 20%, particularly 15%, lower than a control, untreated sample.

In certain embodiments, binding of the immunoconjugate to a PD-1+ cell is not significantly inhibited by PD-1 antagonistic antibodies, particularly by pembrolizumab or nivolumab.

In certain embodiments, binding of the immunoconjugate to recombinant PD-1 does not inhibit binding of recombinant PD-L1 to PD-1.

The inventors used multiple methods to arrive at suitable anti-PD-1 binding domains according to the invention. Firstly, by optimizing the affinity, clinical safety features (e.g. cross-reactivity), and pharmacokinetic properties (e.g.

stability) of non-blocking antibodies to develop antibodies with more favorable clinical properties. A second approach developed suitable novel monoclonal antibodies, by screening hybridomas for non-blocking of pembrolizumab, or nivolumab, as well as multiple parameters such as PD-1 affinity, tissue cross reactivity, heavy and light chain liabilities, and immunogenicity.

In some embodiments of the immunoconjugate according to the invention, the anti-PD-1 binding domain is derived from one of the antibodies disclosed herein as the optimized clones derived from the parent clone z2-XVT458, assigned as z2-XVT458m1-m6 (SEQ ID NOS 057 to 068). In alternative embodiments, the anti-PD-1 binding domain is derived from a clone selected from those disclosed in Tables 14, 15, or 16. Such antibodies show favorable high-affinity binding to PD-1, and cross reactivity to human and primate PD-1 to enable clinical testing in primate models.

In some embodiments of the immunoconjugate according to the invention, the PD1-VH comprises an HCDR1 having the sequence GFTFSINAMT (SEQ ID NO 118), an HCDR2 having the sequence *TISGSGFSTYYADSLKGR* (SEQ ID NO 119), and an HCDR3 having the sequence EVYGDY (SEQ ID NO 120),. In addition, the PD1-VL comprises an LCDR1 having the sequence SGX$_1$SSNIGS(XX)$_2$VF (SEQ ID NO 121; wherein X$_1$ is N, S, Q, or A, and where (XX)$_2$ is NS, QS, SS, or NA), an LCDR2 having the sequence SNNQRPS (SEQ ID NO 122), and an LCDR3 having the sequence AAWDDSLSIWVF (SEQ ID NO 123). In particular embodiments, X$_1$ is selected from the list consisting of S, Q, or A; and X$_2$ is a pair of amino acid residues selected from QS, SS, or NA.

This encompasses PD-1 binding domains tested in the examples with high affinity for PD-1 with modified deamination and glycosylation sites compared to the LCDR1 of the high-affinity anti-PD-1 parent clone 21A08 (see SEQ ID NO 071, 072).

In some embodiments of the immunoconjugate according to the invention, the PD1-VH comprises an HCDR1 having the sequence GFTFSINAMT (SEQ ID NO 118), an HCDR2 having the sequence TISGSGFSTYYADSLKGR (SEQ ID NO 119), and an HCDR3 having the sequence EVYGDY (SEQ ID NO 120). In addition, the PD1-VL comprises an LCDR1 having the sequence SGASSNIGS**QS**VF (SEQ ID NO 124), an LCDR2 having the sequence SNNQRPS (SEQ ID NO 122), and an LCDR3 having the sequence AAWDDSLSIWVF (SEQ ID NO 123). Such embodiments relate to an anti-PD-1 binding domain having the CDR of clone 21A08Ap1, a high affinity anti-PD-1, non-blocking antibody, with little cross-reactivity to other proteins, binding to both human and primate PD-1. In particular embodiments, the full heavy and light chain variable domain are identical to that of 21A8Ap1, wherein the PD1-VH comprises a polypeptide having the sequence SEQ ID NO 085 and the PD1-VL comprises a polypeptide having the sequence SEQ ID NO 086. In more particular embodiments, the PD1-VH consists of SEQ ID NO 085, and the PD1-VL consists of SEQ ID NO 086.

In some embodiments of the immunoconjugate according to the invention, the PD1-VH comprises an HCDR1 having the sequence GFTFSINAMT (SEQ ID NO 118), an HCDR2 having the sequence TISGSGFSTYYADSLKGR (SEQ ID NO 119), and an HCDR3 having the sequence EVYGDY (SEQ ID NO 120). In addition, the PD1-VL comprises an LCDR1 having the sequence SGASSNIG**SS**SVF (SEQ ID NO 125), an LCDR2 having the sequence SNNQRPS (SEQ ID NO 122), and an LCDR3 having the sequence AAWDDSLSIWVF (SEQ ID NO123). Such embodiments relate to an anti-PD-1 binding domain having the CDR of clone 21A08Ap2, a high affinity anti-PD-1, non-blocking antibody, with little cross-reactivity to other proteins, binding to both human and primate PD-1. In particular embodiments, the full heavy and light chain variable domain are identical to that of 21A8Ap2, wherein the PD1-VH comprises a polypeptide having the sequence SEQ ID NO 085 and the PD1-VL comprises a polypeptide having the sequence SEQ ID NO 087. In more particular embodiments, the PD1-VH consists of SEQ ID NO 085, and the PD1-VI consists of SEQ ID NO 087.

In some embodiments of the immunoconjugate according to the invention, the PD1-VH comprises an HCDR1 having the sequence GFTFSINAMT (SEQ ID NO 118), an HCDR2 having the sequence TISGSGFSTYYADSLKGR (SEQ ID NO 119), and an HCDR3 having the sequence EVYGDY (SEQ ID NO 120). In addition, the PD1-VL comprises an LCDR1 having the sequence SGASSNIGS**NA**VF (SEQ ID NO 126), an LCDR2 having the sequence SNNQRPS (SEQ ID NO 122), and an LCDR3 having the sequence AAWDDSLSIWVF (SEQ ID NO 123). Such embodiments relate to an anti-PD-1 binding domain having the CDR of clone 21A08Ap3, a high affinity anti-PD-1, non-blocking antibody binding to both human and primate PD-1. In particular embodiments, the full heavy and light chain variable domain are identical to that of 21A8Ap3, wherein the PD1-VH comprises a polypeptide having the sequence SEQ ID NO 085 and the PD1-VL comprises a polypeptide having the sequence SEQ ID NO 091. In more particular embodiments, the PD1-VH consists of SEQ ID NO 085, and the PD1-VI consists of SEQ ID NO 091.

In certain embodiments, the anti-PD1 antigen binding domain is a variant of 21A08. In particular embodiments thereof, the PD1-VH comprises, or consists of a polypeptide at least ($\geq$) 95%, 98%, 99% similarto SEQ ID NO 085 (VH 21A08), and the PD1-VL comprises, or consists of a polypeptide $\geq$ 95%, 98%, 99% similar to SEQ ID NO 086 (VL 21A08Ap1). In particular embodiments, the PD1-VH comprises, or consists of a polypeptide a polypeptide $\geq$ 95%, 98%, 99% similar to SEQ ID NO 085 (VH 21A08) and the PD1-VL comprises, or consists of a polypeptide $\geq$ 95%, 98%, 99% similar to SEQ ID NO 087 (VL 21A08Ap2). In particular embodiments, the PD1-VH comprises, or consists of a polypeptide a polypeptide $\geq$ 95%, 98%, 99% similar to SEQ ID NO 085 (VH 21A08) and the PD1-VL comprises, or consists of a polypeptide $\geq$ 95%, 98%, 99% similar to SEQ ID NO 091 (VL 21A08Ap3). These variants of 21A08 are characterized by a K$_D$ to PD-1 of $1.0 \times 10^{-9}$ to $1.5 \times 10^{-11}$.

In further embodiments of the immunoconjugate according to the invention, the anti-PD-1 binding domain is derived from the parent clone z2-XVT458, having high affinity for both human and primate PD-1. In addition to the CDR mutations, the sequences of z2-XVT458-m1, z2-XVT458-m3 and z2-XVT458-m6 contain a G57D mutation immediately downstream of the CDRL2, which may contribute to the increased affinity of these clones.

In certain embodiments of the immunoconjugate according to the invention, the PD1-VH comprises an HCDR1 having the sequence NFYIH (SEQ ID NO 127), an HCDR2 having the sequence **R**IYPNYGITAYNQKFKD (SEQ ID NO 128), and an HCDR3 having the sequence GYSYAMDY (SEQ ID NO 129). In addition, the PD1-VL comprises an LCDR1 having the sequence SASQGISGDLN (SEQ ID NO 130), an LCDR2 having the sequence HTS**QR**HS (SEQ ID NO 131), and an LCDR3 having the sequence Q**G**YSKDLLT (SEQ ID NO 132). Such embodiments encompass an anti-PD-1 binding domain having CDR of clone XVT458-Z2-M3. This clone has high affinity binding to human and primate PD-1, as well as little cross-reactivity to other human protein antigens. In particular embodiments, the full heavy and light chain variable domain are identical to that of XVT458-Z2-M3, wherein the PD1-VH comprises, or consists of a polypeptide having the sequence SEQ ID NO 061 and the PD1-VL comprises, or consists of a polypeptide having the sequence SEQ ID NO 062.

In certain embodiments of the immunoconjugate according to the invention, the PD1-VH comprises an HCDR1 having the sequence NFYIH (SEQ ID NO 127), an HCDR2 having the sequence SIYPNYGITAYNQKFKD (SEQ ID NO 133), and an HCDR3 having the sequence GYSYAMDY (SEQ ID NO 129). In addition, the PD1-VL comprises an LCDR1 having the sequence SASQGISGDLN (SEQ ID NO 130), an LCDR2 having the sequence HTS**Q**LHS (SEQ ID NO 134), and an LCDR3 having the sequence Q**G**YSKDLLT (SEQ ID NO 132). Such embodiments encompass an anti-PD-1 binding domain having CDR of clone XVT458-Z2-m6. This clone has high affinity binding to human and primate PD-1, as well as little cross-reactivity to other human protein antigens. In particular embodiments, the full heavy and light chain variable domain are identical to that of XVT458-Z2-m6, wherein the PD1-VH comprises, or consists of a polypeptide having the sequence SEQ ID NO 067 and the PD1-VL comprises, or consists of a polypeptide having the sequence SEQ ID NO 068.

In certain embodiments, the anti-PD1 antigen binding domain is a variant of XVT458. In particular embodiments thereof, the PD1-VH comprises, or consists of a polypeptide $\geq 95\%$, $98\%$, $99\%$ similar to SEQ ID NO 061 (VH XVT458-Z2-M3) and the PD1-VL comprises, or consists of a polypeptide $\geq 95\%$, $98\%$, $99\%$ similar to SEQ ID NO 062 (VL XVT458-Z2-M3). In some embodiments, the PD1-VH comprises, or consists of a polypeptide $\geq 95\%$, $98\%$, $99\%$ similar to SEQ ID NO 067 (VH XVT458-Z2-M6) and the PD1-VL comprises, or consists of a polypeptide $\geq 95\%$, $98\%$, $99\%$ similar to SEQ ID NO 068 (VL XVT458-Z2-M6). These variants of XVT458 are characterized by a $K_D$ to PD-1 of $2.0\times10^{-9}$ to $1.0\times10^{-10}$.

In certain embodiments of the immunoconjugate according to the invention, the affinity constant ($K_D$) of the immunoconjugate for PD-1 is in the range of $4.0\times10^{-9}$ to $1\times10^{-11}$ mol/L, wherein binding affinity is determined using surface plasmon resonance in accordance with the protocol given in Example 2. In particular embodiments the $K_D$ is $2.0\times10^{-9}$ to $1.5\times10^{-11}$ mol/L. In particular embodiments, the anti-PD-1 domain confers very high affinity to PD-1, where the $K_D$ is within the range of $2\times10^{-9}$ to $2\times10^{-11}$ mol/L.

*IL-2 polypeptides and the anti-IL-2 binding domain*

The anti-IL-2 binding domain comprises a heavy chain polypeptide and a light chain polypeptide derived from an anti-IL-2 antibody capable of binding specifically to the human IL-2 protein. An IL-2 polypeptide is covalently linked to amino acid residues of the anti-IL-2 binding domain, optionally by means on one, or two peptide linkers, to provide a single contiguous recombinant polypeptide, providing biased signaling to the dimeric IL-2 receptor.

The anti-IL-2 binding domain comprises firstly, an antibody heavy chain variable domain polypeptide (IL2-VH) having from N' to C' terminus the following domains: a first framework region (IL2VH$_1$), a first complementarity determining region (IL2HCDR1), a second framework region (IL2VH$_2$), a second complementarity determining region (IL2HCDR2), a third framework region (IL2VH$_3$), a third complementarity determining region (IL2HCDR3), and a fourth framework region (IL2VH$_4$). The anti-IL-2 binding domain further comprises an antibody light chain variable domain polypeptide (IL2-VL) having from N' to C' terminus the following domains: a first framework region (IL2VL$_1$), a first complementarity determining region (IL2LCDR1), a second framework region (IL2VL$_2$), a second complementarity determining region (IL2LCDR2), a third framework region (IL2VL$_3$), a third complementarity determining region (an IL2LCDR3), and a fourth framework region (IL2VL$_4$).

Embodiments of an IL-2 polypeptide portion of the immunoconjugate according to the invention include human IL-2, artificial IL-2 variant polypeptides such as Proleukin, or IL-2 muteins as provided in Table 2. The IL-2 polypeptide according to the invention lacks the signal peptide of M1 to S21 of native IL-2 SEQ ID NO 005. An IL-2 polypeptide comprises the following functional regions, a first alpha helix (A), a second alpha helix (B), a third alpha helix (C) and a fourth alpha helix (D) as indicated in Table 2. In some embodiments, the IL-2 polypeptide is a mutein, a recombinant IL-2 protein comprising one or more amino acid substitutions or deletions to modify function, such as reduce binding to CD25. In particular embodiments, the immunoconjugate comprises a mutein selected from those listed in Table 2. A mutein, or IL-2 variant polypeptide according to the invention is >90%, particularly 95%, 98% similar to SEQ ID NO 005 and has equivalent biological function to SEQ ID NO 005. Non-limiting examples of variants and muteins are disclosed as SEQ ID NO 006 and 007. Biological function for an IL-2 polypeptide is defined according to the invention as an affinity for the dimeric IL-2 receptor in the range of the $K_D$ of the immunoconjugate for the dimeric IL-2 receptor is in the range of 500 nM to 0.1 nM, particularly 100 nM to 0.1 nM, more particularly 10 nM to 0.1 nM, still more particularly wherein the $K_D$ is in the range of 1 nM to 0.1 nM, as measured by surface plasmon resonance (SPR).

*Circularly permuted IL-2 polypeptides*

In particular embodiments of the immunoconjugate according to the invention, the IL-2 polypeptide is a circularly permuted IL-2 (IL2CP), having functional domains reordered relative to their domain position in the wildtype sequence of SEQ ID NO 005, such as the examples provided in in Table 3. Particular embodiments of an IL-2 polypeptide starting sequence to which circular permutation can be applied are WT SEQ ID NO 005, SEQ ID NO 006 or the muteins listed in the Table 2.

A circularly permuted IL-2 polypeptide is created by "opening" an IL-2 polypeptide sequence, such as SEQ ID NO 005 or 006, to create a new N' terminus and C' terminus, as well as joining natural N' and C' terminal amino acid residues (see SEQ ID NO 010 to 013 for examples of the resulting circularly permuted interleukin sequence). This provides a reordered IL2CP polypeptide retaining the tertiary structure of human IL-2 (see for example WO2013184942A1). In some embodiments, the IL-2 polypeptide is opened between two consecutive amino acids. In other embodiments, the IL-2 polypeptide is opened between two positions within one region (thus potentially excising 1, or several amino acids of the IL-2 polypeptide as in the structures disclosed in WO2013184942A1). In certain embodiments, the new IL2CP N- and C- termini are amino acids no more than 2 amino acids apart in the base IL-2 polypeptide sequence. In more particular embodiments, the IL2CP C- and N- termini are adjacent residues in the human IL-2 polypeptide or mutein, such that the IL2CP comprises all amino acid residues of the base IL-2 polypeptide sequence, with none excised.

In particular embodiments of the immunoconjugate, the anti-IL-2 binding domain is joined to an IL2CP in which an N-terminal amino acid of the human IL-2 polypeptide or variant thereof, particularly an N-terminal amino acid selected from A21, P22, T23, S24, S25, or S26, is joined to a C-terminal amino of the human IL-2 polypeptide or variant thereof selected from T150, L152, and T153. In more particular embodiments, the IL-2 residue P22, is joined to T153.

Importantly, the IL2CP it is opened at a location that does not disrupt an alpha helix structure. The inventors identified such regions between the C and B helix, the A and B helix, and between the C and D helix. In particular embodiments, both the new N-terminus, and the C-terminus located within the region V89 to D104, particularly S95 to N97, more particularly K96 and N97. This provides an IL2CP cut between the C/B, where the alpha helices of the IL2CP are in the order CDAB. In some embodiments, both the new N-terminus, and the C-terminus located within the region G47 to E72, particularly L56 to position E72, more particularly K63 to F64. This provides an IL2CP cut between the A/B, where the alpha helices of the IL2CP are in the order BCDA. In some embodiments, both the new N-terminus, and the C-terminus located within the region G118 to I134, particularly between S119 to A132. This provides an IL2CP cut between the C/D, where the alpha helices of the IL2CP are in the order DABC.

*Fusion of anti-IL-2 binding domain and IL-2 polypeptides*

In the immunoconjugate according to the invention, one of the antibody variable domains within the heavy chain or the light chain of the anti-IL-2 antigen binding domain is joined, optionally by means of one or two peptide linkers, to an IL-2 polypeptide. Together, the anti-IL-2 binding domain joined to the IL-2 polypeptide, delivers biased IL-2 signaling to the dimer IL-2 receptor expressed on exhausted CD8 T cells. In some embodiments, the IL2CP N-terminus or the L2CP C-terminus is covalently linked, optionally by means of one or two peptide linkers to the IL2-VL, or the IL2-VH. In some embodiments, the IL2CP N-terminus or the L2CP C-terminus is covalently linked, optionally by means of one, or two peptide linkers to the IL2-VL, and the IL2-VH, in other words, the IL2CP is embedded within the IL2-VL or IL2-VH.

In some embodiments of the immunoconjugate according to invention, both the IL2CP N' terminus and IL2 CP C' terminus are covalently linked, optionally by means of one, or two peptide linkers, to amino acid residues within a domain selected from the list consisting of IL2HCDR1, IL2HCDR2, IL2VH$_3$, IL2HCDR3, IL2LCDR1, IL2LCDR2, IL2VL$_3$ or IL2LCDR3. Crystal structure analysis by the inventors suggests these are particularly helpful sites for embedding an IL2CP to ensure optimal placement of the cytokine with reference to an anti-IL-2 binding domain, preserving biased IL-2 signaling signals to IL-2 receptors.

In some embodiments of the immunoconjugate according to invention, the anti-IL-2 binding domain is fused to an IL2CP at least (>) 98% similar to SEQ ID NO 010, the IL2CP CDAB variant 1 validated in Example 1. In some embodiments of the immunoconjugate according to invention, the anti-IL-2 binding domain is fused to an IL2CP at least (>) 98% similar to SEQ ID NO 011, the IL2CP CDAB variant 2 validated in Example 1. In these embodiments, the IL2CP N-terminus and IL2CP C-terminus are covalently linked, directly, or by means of one, or two peptide linkers, to amino acid residues within a domain selected from the list consisting of IL2LCDR1, IL2LCDR2, IL2HCDR1, or IL2HCDR3, particularly IL2LCDR1, or IL2HCDR3. In particular embodiments, a CDAB IL2CP polypeptide is embedded within IL2LCDR1.

In some embodiments of the immunoconjugate according to invention, the anti-IL-2 binding domain is joined to an IL2CP at least (>) 98% similar to SEQ ID NO 012, the IL2CP variant 3 BCDA. In these embodiments, the IL2CP N-terminus and IL2CP C-terminus are covalently linked, optionally by means of one or two peptide linkers, to amino acid residues within a domain selected from the list consisting of IL2LCDR1, IL2LCDR3, IL2HCDR1, IL2HCDR2, IL2VH$_3$. In a particular embodiment, the IL2CP N-terminus and IL2CP C-terminus are linked to IL2HCDR2. An IL2CP in the BCDA format may also be joined to the anti-IL-2 binding domain at the end of IL2VL$_4$ by means of a flexible linker.

In some embodiments of the immunoconjugate according to invention, the anti-IL-2 binding domain is joined to an IL2CP at least (>) 98% similar to SEQ ID NO 013, the IL2CP variant 3 DABC. In these embodiments, the IL2CP N-terminus and IL2CP C-terminus are preferably covalently linked, optionally by means of one, or two peptide linkers, to amino acid residues within a domain selected from the list consisting of IL2HCDR2 and IL2VH$_3$.

In particular embodiments of the immunoconjugate according to the invention, no amino acids "0", or 1, 2, 3, or 4, particularly 2, consecutive amino acids within a domain selected from the list consisting of IL2HCDR1, IL2HCDR2, IL2VH$_3$, IL2HCDR3, IL2LCDR1, IL2LCDR2, IL2VL$_3$, or IL2LCDR3, have been replaced with a polypeptide consisting of, listed from N- to C- direction: a first peptide linker, an IL2CP as described in the preceding five paragraphs, and a second peptide linker. In reference to this aspect of the invention "0" means the sequences is inserted into the IL2-VH or IL2-VL region, but no amino acids from the anti-IL-2 binding domain antibody portion are lost; "1" means the IL2CP is inserted into the place where the one lost amino acid was, "2" is the same but two amino acids from the IL-2-VH or VL were lost, or replaced by the IL2CP, and so on.

In particular embodiments of the immunoconjugate according the invention, it comprises an IL2CP embedded within the IL-2-VH, or the IL-2-VL, flanked, or joined, at each end by a short peptide linker. In more particular embodiments, both the first and second peptide linkers are 1-20 amino acids in length. In still more particular embodiments both the first and second peptide linkers are between 2 and 7 amino acids in length. In further particular embodiments of the immunoconjugate according to the invention, any peptide linker flanking the IL2CP is comprised of glycine (G), or G and serine (S) residues. In more particular embodiments, the peptide linker joining the C- or N-terminal residue of the IL2 polypeptide to amino acids of the IL-2VH or IL-VL has a sequence selected from the sequences assigned as SEQ ID NO 014-024:

SEQ ID NO 014: Peptide linker (artificial): G
SEQ ID NO 015: Peptide linker (artificial): GG
SEQ ID NO 016: Peptide linker (artificial): GGG
SEQ ID NO 017: Peptide linker (artificial): GGGG
SEQ ID NO 018: Peptide linker (artificial): G$_4$S
SEQ ID NO 019: Peptide linker (artificial): G$_4$SG
SEQ ID NO 020: Peptide linker (artificial): G$_4$SGG
SEQ ID NO 021: Peptide linker (artificial): (G$_4$S)$_2$GGG
SEQ ID NO 022: Peptide linker (artificial): (G$_4$S)$_2$GGGG
SEQ ID NO 023: Peptide linker (artificial): (G$_4$S)$_3$GGGG
SEQ ID NO 024: Peptide linker (artificial): (G$_4$S)$_4$

*N-terminal linked IL-2 or IL2CP*

In alternative embodiments of the immunoconjugate according to the invention, the IL-2 polypeptide (optionally an IL2CP) is covalently linked by means of a peptide linker to an N-terminal amino acid residue of the IL2VL$_4$ domain, or to an N-terminal amino acid residue of the IL2VH$_4$ domain. In particular embodiments, the N-terminal peptide linker is between 10 to 30, more particularly 21 or 22 amino acids in length. Cytokines can be linked to antibody binding domains when combined with medium linkage peptides, allowing flexibility of positioning of the cytokine with the antibody binding domain, however longer peptide linkers can allow *trans* interactions between molecules which can lead to formation of aggregates.

*Receptor-biased IL-2 signaling*

In particular embodiments of the immunoconjugate according to the invention, the immunoconjugate is characterized in that the K$_D$ of the immunoconjugate for the dimeric IL-2 receptor (comprising CD122 and CD132) is in the range of 500 nM to 0.1 nM, 100 nM to 0.1 nM, 10 nM to 0.1 nM, particularly wherein the $K_D$ is in the range of 1 nM to 0.1 nM. Binding affinity according to this aspect of the invention is to be determined using surface plasmon resonance. Binding of Fab fragment of representative compound comprising an IL-2-anti-IL-2-binding domain fusion according to the invention (QTY065) to CD122:CD132 is 0.322 nM, as measured by SPR in the examples at 200 nM. A further aspect of receptor biased binding can be characterized as the immunoconjugate having no detectable binding to CD25, as measured by SPR at 200 nM.

*Anti-IL-2 binding domains*

The designation of CDR provided in embodiments of the anti-IL-2 binding domain provided herein are based on an uninterrupted antibody sequence, and is understood that the sequence may be interrupted as defined herein, by an IL2CP fused within the IL2-VH, or the IL2-VL. Otherwise, it is clear that alignment to the canonical CDR sequences would not be recognized. Therefore, the term CDR according to the invention encompasses not just canonical CDR amino acids, but may be a longer recombinant polypeptide in which those residues aligning to the canonical CDR residues are interrupted by an IL2CP, and optionally, peptide linkers joining the antibody binding region of the immunoconjugate to the IL-2 polypeptide domain.

In certain embodiments of the immunoconjugate according to the invention, the IL2-VH is >95%, particularly more than >98%, or 99% identical to SEQ ID NO 043 (VH Antibody A), and the IL2-VL is >95% particularly more than >98%, or 99% identical to SEQ ID NO 042 (VL Antibody A), and has a similar biological function to Antibody A (and with the proviso that the IL-2 polypeptide, or peptide linkers optionally embedded in one of said variable chains according to e.g. claim 2 are excluded from the amino acid alignment/sequence identity). According to these embodiments, the affinity constant ($K_D$) of an antibody characterized by the IL-2VH and the IL2-VL for an IL-2 polypeptide having SEQ ID NO 006 (Proleukin) is in the range of 500 nM to 2 nM, particularly 270 nM to 2 nM, more particularly about 2 nM. The determination of this affinity constant ($K_D$) refers to the interaction of the corresponding free antibody as employed as an anti-IL-2 binding domain according to the invention, and free IL-2 (not the immunoconjugate wherein those two features are linked).

In certain embodiments of the immunoconjugate according to the invention, the anti-IL-2 antigen binding domain comprises, or consists of, an IL2-VH having sequence SEQ ID NO 043 (Antibody A VH), associated with an IL2-VL selected from SEQ ID NO 025 to 031. Such embodiments encompass the LCDR1 IL2CP-embedded Antibody A structures EAD409, XFO227, QTY065, FJC828, PG035, DRV470, XUB802. In particular embodiments the anti-IL-2 binding domain comprises, or consists of SEQ ID NO 043 and SEQ ID NO 027.

In certain embodiments of the immunoconjugate according to the invention, the anti-IL-2 antigen binding domain comprises, or consists of, an IL2-VL having sequence SEQ ID NO 042 (Antibody A VL), associated with an IL2-VH selected from SEQ ID NO 046, or 047. Such embodiments relate to the Antibody A-based CDRH3 (VBE401) or CDRH2 (LI707) IL2CP-embedded structures, each comprising alternative validated IL2CP sequences.

In further embodiments of the immunoconjugate according to the invention, the IL2-VH is >95%, particularly more than >98%, or 99% identical to SEQ ID NO 044 (VH Antibody B), and the IL2-VL is >95 particularly more than >98%, or 99% identical to SEQ ID NO 050 (VL Antibody B). In such embodiments, the affinity constant ($K_D$) of an antibody characterized by the IL-2VH and the IL2-VL for an IL-2 polypeptide having SEQ ID NO 006 (Proleukin) is in the range of 500 nM to 2 nM, particularly 270 nM to 2 nM.

In certain embodiments of the immunoconjugate according to the invention, the anti-IL-2 antigen binding domain comprises, or consists of, an IL2-VH having sequence SEQ ID NO 044 (Antibody B VH), associated with an IL2-VL selected from SEQ ID NO 032 to 036. These embodiments relate to IL2CP embedded into LCDR1 of Antibody B, EPK959, GYG794, DRO069, or ECV200, and BFC885. These also showed little binding to CD25 as measured by SPR (Table 5). These validated alternatives of IL2CP composition and linkage embedding, deliver biased signaling to the dimeric IL-2 receptor according to the invention.

*Fc portions*

In certain embodiments of the immunoconjugate according to the invention, the immunoconjugate comprises an Fc portion, conferring extended half-life in vivo. In particular embodiments, the Ig Fc is an IgG Fc portion.

In certain embodiments of the immunoconjugate, the IgG Fc portion is characterized by the presence of one or more modifications to constant regions of the heavy chains to enhance correct heavy chain pairing. In particular embodiments, the modifications are selected from the following knob and hole paired mutations to enhance heavy chain pairing:

- Knob: S354C, T366W and Hole: Y349C, T366S, L368A, Y407V;
- Knob: T366Y, and Hole Y407T;
- Knob: Y349C T366W, and Hole: S354C, T366S, L368A, Y407V;

- Knob: T366W, and Hole: Y407A, T366S, L368A.

In certain embodiments of the immunoconjugate according to the invention, the IgG Fc portion is characterized by the presence of one or more modifications to constant regions of the heavy chains to reduce effector function of the Fc portion. In particular embodiments, said modification, or modifications are selected from L234A, L235A (LALA), L234A, L235A, P329G (LALA-PG), L234A, L235A, P329A (LALA-PA), N297A, N297Q, N297G and D265A, N297G (DANG). In particular embodiments, the immunoconjugate comprises P329A (LALA-PA).

*Immunoconjugate formats*

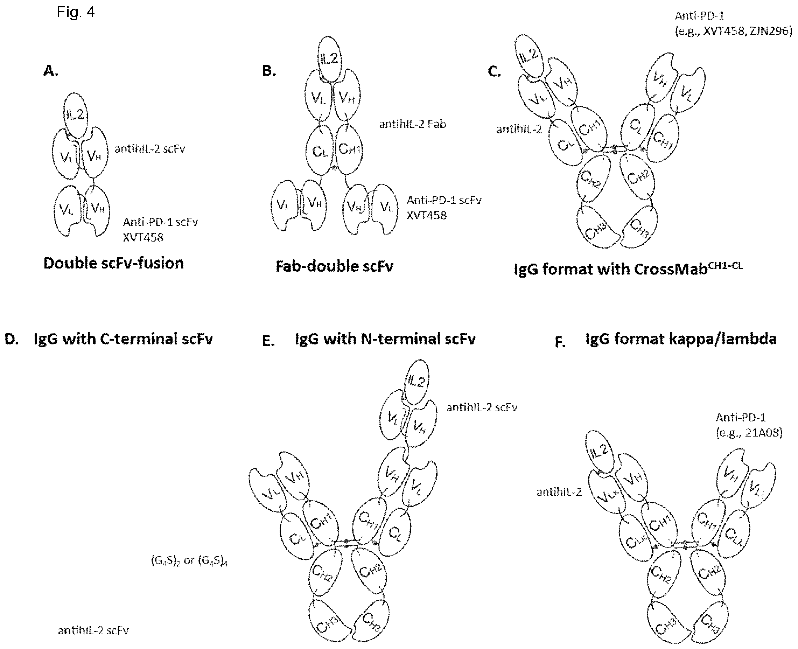

In particular embodiments of the immunoconjugate according to the invention, the immunoconjugate is a heterotetrameric IgG consisting of a first heterodimer comprising the anti-PD-1 binding domain, and a second heterodimer comprising the anti-IL2 binding domain. This format is illustrated in Figure 4, and includes such formats as a heterotetrameric kappa/lambda IgG format, and the Crossmab format (Fig. 4C, WO2009 080253).

In particular embodiments of the immunoconjugate according the invention, the immunoconjugate is a heterotetrameric kappa/lambda IgG format, having an anti-PD-1 binding domain is characterized by a lambda light chain, for example derived from the 21A08 antibody clones listed herein (Fig. 4F).

In certain embodiments of the immunoconjugate according to the invention, the immunoconjugate comprises, or consists of the polypeptides having the sequences SEQ ID NO 111, SEQ ID NO 095, SEQ ID NO 112, and SEQ ID NO 052 (NZA596).

In certain embodiments of the immunoconjugate according to the invention, the immunoconjugate comprises, or consists of the polypeptides having the sequences SEQ ID NO 094, SEQ ID NO 095, SEQ ID NO 100, and SEQ ID NO 052 (XWY176).

In certain embodiments of the immunoconjugate according to the invention, the immunoconjugate comprises, or consists of the polypeptides having the sequences SEQ ID NO 097, SEQ ID NO 098, SEQ ID NO 100, and SEQ ID NO 052 (GQM289).

In certain embodiments of the immunoconjugate according to the invention, the immunoconjugate comprises, or consists of the polypeptides having the sequences SEQ ID NO 117, SEQ ID NO 104, SEQ ID NO 112, and SEQ ID NO 052 (BGY642).

In alternative embodiments of the immunoconjugate according to the invention, the immunoconjugate is an immunoglobulin ScFv format as illustrated in Fig. 4D and 4E. This format comprises an anti-PD1 antibody comprising a first and a second anti-PD-1 binding domain. Both the first and the second binding domain are characterized by an antibody heavy chain comprising a PD1-VH and an antibody light chain comprising a PD1-VL as specified in the section *Anti-PD1 binding domains*. The format further comprises an anti-IL-2 ScFv comprising an anti-IL-2 binding domain as specified in the section *anti-IL-2 binding domains*. In the immunoglobulin ScFv format, the C-terminal residue of the anti-IL-2 ScFv IL2-VH, or the IL2-VL domain is linked via a peptide linker to the N-terminal residues of the heavy chain, or the light chain of the anti-PD1 antibody. In particularly embodiments, said peptide linker is 10 to 30 amino acids in length. In still more particular embodiments, said peptide linker has the sequence (G4S)2 or SEQ ID NO 024.

In certain embodiments of the immunoconjugate according to the invention, the immunoconjugate comprises, or consists of the polypeptides having the sequences SEQ ID NO 094, 095, and 096. In certain embodiments of the immunoconjugate according to the invention, the immunoconjugate comprises, or consists of the polypeptides having the sequences SEQ ID NO 097, 098, and 099. In certain embodiments of the immunoconjugate according to the invention, the immunoconjugate comprises, or consists of the polypeptides having the sequences SEQ ID NO 105, 106, and 107. In certain embodiments of the immunoconjugate according to the invention, the immunoconjugate comprises, or consists of the polypeptides having the sequences SEQ ID NO 108, 109, and 110. In certain embodiments of the immunoconjugate according to the invention, the immunoconjugate comprises, or consists of the polypeptides having the sequences SEQ ID NO 113, 106, and 114. In certain embodiments of the immunoconjugate according to the invention, the immunoconjugate comprises, or consists of the polypeptides having the sequences SEQ ID NO 115, 109, and 116. These can all be represented by the format of Fig. 4E.

*Further aspects of the invention*

Another aspect of the invention relates to an isolated nucleic acid encoding the immunoconjugate according to any one of the embodiments of the immunoconjugate aspect of the invention. Another aspect of the invention relates to an expression vector comprising said isolated nucleic acid. The invention further encompasses a host cell comprising said nucleic acid or said expression vector.

Another aspect of the invention relates to a combination medicament comprising

i) an immunoconjugate as specified in any one of the aspects or embodiments of the invention herein, and
ii) an anti-PD-1 antagonist antibody.

In particular embodiments the anti-PD1 antagonist antibody is selected from the list consisting of nivolumab, pembrolizumab, dostarlimab, sintilimab, tislelizumab, cemiplimab, cetrelimab, sasanlimab. In more particular embodiments, the antibody is nivolumab or pembrolizumab.

The invention is further illustrated by the following examples and figures, from which further embodiments and advantages can be drawn. These examples are meant to illustrate the invention but not to limit its scope.

## Description of the Figures

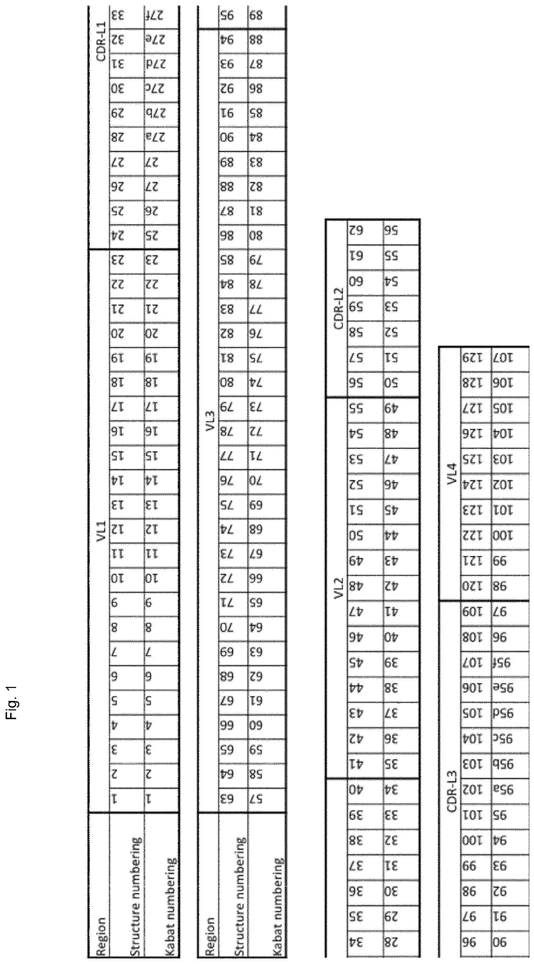

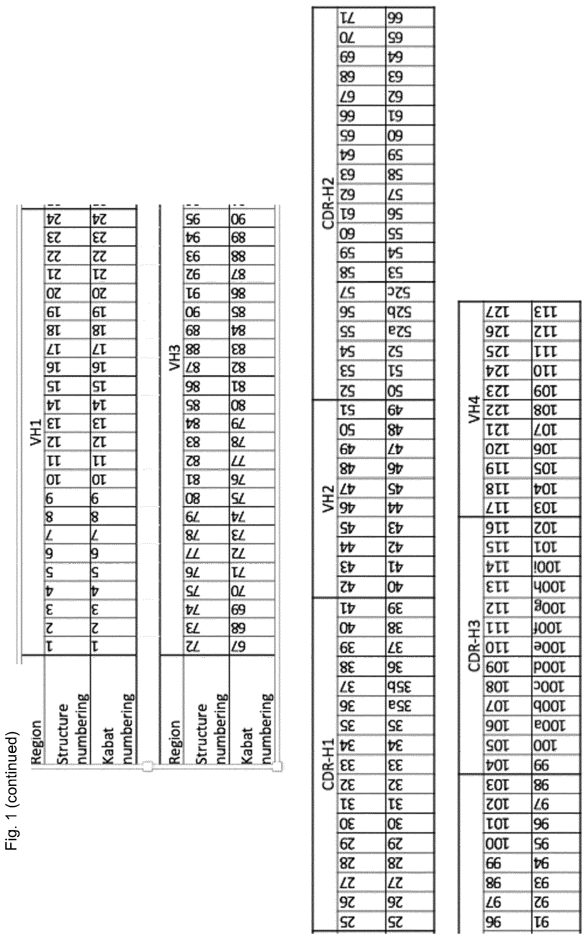

Fig. 1    shows labelling of antibody residues according to accepted formats with standard numbering and Kabat numbering systems.

Fig. 2    shows how IL2-CP formats can be varied to suit embedding in various variable domain sites. (Left) a figure of the IL-2 with the correct orientation for binding to Antibody A or B (Middle) a figure of LCDR1 embedded CDAB IL-2CP in Antibody A, B or C (right) HCDR3 embedded BCDA IL-2CP.

Fig. 3    SPR sensograms (Response Units, RU over Time in seconds) of PD-1 pre-mixed with antibodies or Buffer run as analyte on immobilized PD-L1.

Fig. 4    shows proof of principal formats with various PD-1 binding moiety arrangement, including A. a double scFv-fusion, B. a Fab-double scFC, C. a heterotetrameric IgG (IgG CrossMab), aD-E.two bivalent PD-1 IgG antibodies, with two linkage positions of an anti-hIL-2 ScFv F. a heterotetrameric IgG (kappa/lambda).

Fig. 5    shows cell proliferation analyzed by flow cytometry, detecting Ki67+ cells in CD8 T cells, NK cells and Treg cells. Bispecific compounds administered i.v. at 0.2mg/kg to wt C57BL/6 mice. Blood samples were taken before the injection and on days 3 and 6 after compound administration.

## Examples

### Example 1: Design and production of anti-IL-2 antibody IL-2 cytokine fusion proteins

Antibodies binding to IL-2 were derived, isolated and structurally characterized according to methods well known to a person skilled in the art. Antibody A (HC SEQ ID NO 001, LC SEQ ID NO 002) is a high-affinity anti-IL-2 antibody and Antibody B (HC SEQ ID NO 003, LC SEQ ID NO 004) is a low-affinity anti-IL-2 antibody. Antibody C has no IL-2 affinity and has been included as a control. The genes encoding for VL-CL (light chain) and VH-CH1-CH2-CH3 (heavy chain) were cloned into the mammalian expression vector pcDNA3.4 into separate plasmids. The antibodies were produced using transient gene expression in Expi293 cells (Gibco, A14527) following standard protocols provided by the vendor. Plasmid DNA (HC/LC ratio 1:2 w/w) was transfected using ExpiFectamine™ 293 Reagent. The cells were maintained at 37°C, 8% CO2 in an orbital shaker (150rpm) for six days. The antibodies were purified to homogeneity from the supernatant by Protein A Chromatography (MabSelect™ SuRe™, GE17-5438-01). Quality control of the proteins was performed by SDS-PAGE (NuPAGE™ 4-12% Bis-Tris Protein Gels , ThermoFisher) and analytic size exclusion chromatography (SEC) (GE lifesciences, Superdex 200 increase 10/300). Yields and purity are reported in Table 1. All antibodies were obtained as pure protein product, eluting as single peak in SEC-HPLC analysis.

Binding to IL-2 was first tested by ELISA using a serial dilution of antibodies on coated IL-2. Recombinant human IL-2 was coated on Maxisorp ELISA plates (Invitrogen, 44-2404-21) at 5μg/ml, 4°C overnight. After 2 hours blocking, antibodies A, B and C were incubated in a serial dilution starting from 10 μg/ml in assay buffer. The antibodies were detected with anti-human IgG-Peroxidase (Sigma, A0170). After addition and blockade of the chemiluminescent substrate, absorption was read ($A_{450}$-$A_{570}$ at plate reader, Spectramax iD3). $EC_{50}$ values were obtained by plotting Absorbance vs. log(Concentration) and sigmoidal 4-PL fitting using GraphPad Prism (Table 1). Only Antibody A displayed binding to IL-2 in ELISA and could be detected on the Fc-portion by an anti-human IgG antibody coupled to HRP. Binding affinities to recombinant human IL-2 (rhIL-2) of Antibodies A, B and C of the present invention were then measured using more sensitive biolayer interferometry (BLI) methodology. Antibodies were immobilized on amine reactive (2nd Generation) sensors (ForteBio, 18-5092). Association (600s) and dissociation (900s) of a dilution series of recombinant IL-2 (Aero Biosystems, IL2-H4113) were measured on the antibody-coated biosensors on an Octet -System (Octet RED, ForteBio). $K_D$ values were obtained by fitting the kinetics data with the ForteBio Data Analysis Software (8.2). This assay confirmed Antibody A binds rhIL-2 with high affinity. Binding of Antibody B could not be measured by ELISA, but a weak affinity constant could be determined by BLI. Antibody C did not show binding to rhIL-2 up to concentrations ≥1000 nM (Table 1).

Table 1 Production yields from transient expression, purity and binding affinities to recombinant hIL-2 measured by ELISA or BLI.

| Antibodies | Yield (mg/ml) | Purity by SEC-HPLC (%) | $EC_{50}$ to IL-2 by ELISA (nM) | $K_D$ to IL-2 by BLI (nM) |
|---|---|---|---|---|
| A | 193.9 | 95.8% | 0.37 | 2.1 |
| B | 282.4 | 98.9% | - | 269.8 |
| C | 453.5 | 98.5% | - | - |

Fusion proteins comprising an IL-2 polypeptide, or an IL-2 mutein comprising amino acid substitutions with favorable pharmacokinetic properties (Table 2), have previously been demonstrated to provide IL-2 signaling when conjugating the cytokine to an antibody chain by C-terminal linkage to an antibody variable chain region (see for example, WO2018184964A1; Deak LC. et al. 2022, Nature 610:161; Gutbrodt KL. 2013 Sci. Trans. Med. 5:201; Gillies SD. (1992) PNAS 89(4):1428), or N-terminal linkage to an anti-IL-2 antibody (WO2017122130A1).

Table 2. IL-2 polypeptides

| SEQ ID NO | Cytokine or mutein |
|---|---|
| 005 | Human IL-2 (hIL2, Uniprot P60568) ; Numbering 1-153 |
| 006 | Proleukin® (aldesleukin) |
| 007 | no-alpha mutein |
| 008 | WO2012/107417A1 IL2 mutein |
| 009 | IL-2 superkine |

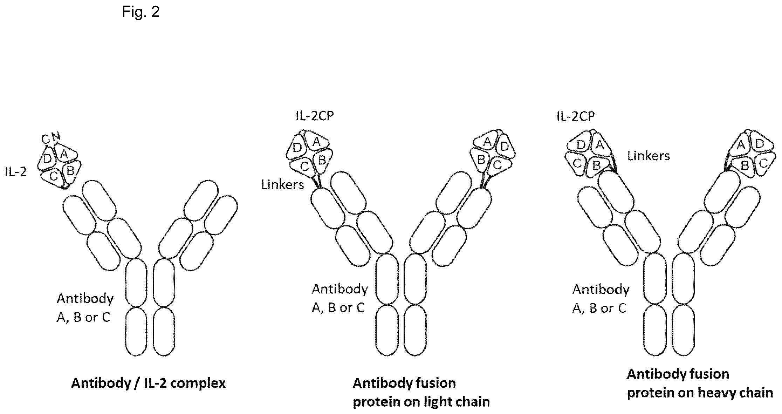

The inventor's assessed an alternative format, analyzing the structure of IL-2 in complex with the antibody NARA1 (RCSB Protein data bank 5LQB) from which humanized Antibody A and Antibody B were derived, to guide the embedding or fusion of IL-2 to preserve orientation of the cytokine relative to the antibody and similar receptor signaling qualities (Arena-Ramires et al. 2016 Sci. Trans. Med. 8:367). Based on the crystal structure of the NARA1/IL-2 complex, possible sites for embedding were identified by checking connecting strands between the different alpha helix domains (assigned here as A-D) and their proximity to CDR or framework regions on the VH or VL of the antibody. Various combinations of circularly permuted IL-2 (IL-2CP) were designed, to retain the cytokine's tertiary structure and orientation with respect to natural binding to the antibody, when fused to the indicated heavy chain or light chain variable regions (Table 3, Figure 2). Depending on the juncture site, the IL-2 needs to be permuted differently, in order to maintain the same orientation on the antibody. To create a circular permutation with the IL-2 helix domains CDAB format, the crystal structure suggested the region V89 to D104 (referring to the sequence of Proleukin) between the Band C helix could be opened to maintain key tertiary structures. To create a circular permutation with the IL-2 helix domains BCDA format, the crystal structure suggested the region G47 to E72 between the A and B helix could be opened. To create a circular permutation with the IL-2 helix domains DABC format, the crystal structure suggested the region G118 to I134 between the C and D helix could be targeted.

Table 3. Circularly permuted IL-2 polypeptides and optimal linkage sites

| SEQ ID NO | | | Fusion |
|---|---|---|---|
| 10 | Circularly permuted Proleukin variant 1 | NFHLRPRDLISNINVIVLELKGSETTFMCE YADETATIVEFLNRWITFSQSIISTLTPTS SSTKKTQLQLEHLLLDLQMILNGINNYKNP KLTRMLTFKFYMPKKATELKHLQCLEEELK PLEEVLNLAQSK | CDRL1, CDRL2, CDRH1, CDRH3, |
| 11 | Circularly permuted Proleukin variant 2 | FHLRPRDLISNINVIVLELKGSETTFMCEY ADETATIVEFLNRWITFSQSIISTLTPTSS STKKTQLQLEHLLLDLQMILNGINNYKNPK LTRMLTFKFYMPKKATELKHLQCLEEELKP LEEVLNLAQSK | CDRL1, CDRL2, CDRH1, CDRH3 |
| 12 | Circularly permuted Proleukin variant 3 | FYMPKKATELKHLQCLEEELKPLEEVLNLA QSKNFHLRPRDLISNINVIVLELKGSETTF MCEYADETATIVEFLNRWITFSQSIISTLT PTSSSTKKTQLQLEHLLLDLQMILNGINNY KNPKLTRMLTFK | CDRL1, CDRL2 CDRL3 CDRH1, CDRH2, FW3VH, CDR VL-N terminal |
| 13 | Circularly permuted Proleukin variant 4 | ATIVEFLNRWITFCQSIISTLTPTSSSTKK TQLQLEHLLLDLQMILNGINNYKNPKLTRM LTFKFYMPKKATELKHLQCLEEELKPLEEV LNLAQSKNFHLRPRDLISNINVIVLELKGS ETTFMCEYADET | CDRH2, FW3VH |

Representative IL-2 fusion proteins were then designed according to some embodiments of the present invention comprising Antibodies A, B or C joined to IL-2 either directly, or by means of linkers of various lengths as set out in Table 4.

The LCDR1 of the IL-2 Antibody A Kabat residues Y27d-D30 (or Y31-D34 structure numbering), and the region connecting IL-2 helix B and helix C between residue S95 and N97 were identified as promising regions for further engineering. The LCDR1 of each Antibody A was opened between Y27d and D30 numbered according to Kabat definition, to provide new C-terminal residue at LCDR1 Y27d, and a new N-terminal LCDR1 D30. LCDR1 residue Y27d was joined to the N-terminus of an IL-2CP opened between K96 and N97 (SEQ ID NO 10). The C-terminus of IL-2CP was joined to the LCDR1 residue 30 by the Kabat definition, such that residues Q28 and G29 of LCDR1 of the antibody were replaced by the circularized IL-2CP SEQ ID NO 10, joined directly, or by means of peptide linkers of various lengths. This embedding was repeated for equivalent amino acid residues with LCDR1 of the lower affinity IL-2 Antibody B, and Antibody C having no affinity for IL-2 (Table 4).

Further constructs were designed to validate an alternative IL-2CP insertion in the HDCR3, or HCDR2 region. VBE401 was developed using SEQ ID NO 011, an IL-2 opened between N97 and K96, inserting the cytokine (removing N97 and leaving F96 as new N-terminal end). SEQ ID NO 011 was inserted between residues E98 and G99 in the HCDR3 of Antibody A preceded by the linker GGG, and followed by the linker GGG. In addition, insertion of an IL-2CP into HDRC2 was tested. A circularly permuted IL-2 was created by opening the sequence between K63 and F64, and fusing the original N- and C-termini to provide SEQ ID NO 012. SEQ ID NO 012 was inserted between Antibody A HDCR2 residues G53 and S54, preceded by the linker GG, and followed by the linker GGG as described above (LIZ707).

For each structure (Table 4), the genes encoding VL-(IL-2)-CL, VL-CL, VH-CH1-CH2-CH3, VH-(IL-2)-CH1-CH2-CH3 were cloned into the mammalian expression vector pcDNA3.4 into separate plasmids. The antibody IL-2 fusion proteins were produced using transient gene expression in Expi293 cells as described for antibodies A, B and C above. The purity of all constructs was high (>95%), eluting as single peak in SEC-HPLC analysis (Table 5).

Table 4. Antibody-IL-2 fusion proteins, with IL-2CP fusion site on the respective CDR of antibodies A, B or C. IL-2CP sequence is embedded within the variable domain of the heavy or light chain of the antibody as indicated. All heavy chains constant regions were SEQ ID NO 048, all light chain constant regions were SEQ ID NO 049.

| Compound | IL-2 fusion site, Antibody clone | IL2-CP (SEQ ID NO) | Linker 1 (SEQ ID NO) | Linker 2 (SEQ ID NO) | VL (SEQ ID NO) | VH (SEQ ID NO) |
|---|---|---|---|---|---|---|
| EAD406 | CDR-L1, A | 010 | 0 AA | 1 AA (014) | 025 | 043 |
| XFO227 | CDR-L1, A | 010 | 2 AA (015) | 3AA (016) | 026 | 043 |
| QTY065 | CDR-L1, A | 010 | 3 AA (016) | 4 AA (017) | 027 | 043 |
| FJC828 | CDR-L1, A | 010 | 4 AA (017) | 5 AA (018) | 028 | 043 |
| PGO345 | CDR-L1, A | 010 | 6 AA (019) | 7 AA (020) | 029 | 043 |
| DRV470 | CDR-L1, A | 010 | 13 AA (021) | 14AA (022) | 030 | 043 |
| XUB802 | CDR-L1, A | 010 | 19 AA (023) | 20 AA (024) | 031 | 043 |
| EPK959 | CDR-L1, B | 010 | 0 AA | 1 AA (014) | 032 | 044 |
| GYG794 | CDR-L1, B | 010 | 2 AA (015) | 3 AA (016) | 033 | 044 |
| DRO069 | CDR-L1, B | 010 | 3 AA (016) | 4 AA (017) | 034 | 044 |
| ECV200 | CDR-L1, B | 010 | 6 AA (019) | 7 AA (020) | 035 | 044 |
| BFC885 | CDR-L1, B | 010 | 13 AA (021) | 14AA (022) | 036 | 044 |
| LPT269 | CDR-L1, C | 010 | 0 AA | 1 AA (014) | 037 | 045 |
| DXM339 | CDR-L1, C | 010 | 2 AA (015) | 3AA (016) | 038 | 045 |
| FUE433 | CDR-L1, C | 010 | 3 AA (016) | 4 AA (017) | 039 | 045 |
| LQM346 | CDR-L1, C | 010 | 6AA (019) | 7 AA (020) | 040 | 045 |
| GLK754 | CDR-L1, C | 010 | 13 AA (021) | 14AA (022) | 041 | 045 |
| VBE401 | CDR-H3, A | 011 | 3 AA (016) | 3 AA (016) | 042 | 046 |
| LIZ707 | CDR-H2, A | 012 | 2 AA (015) | 3 AA (016) | 042 | 047 |

*Immunoconjugate interactions with the CD132-CD122 heterodimeric receptor*

The anti-IL-2 antibody clone 5344, binding on the CD122 binding site on IL-2, was used in order to determine correct folding of the IL-2 portion of the fusion proteins. The $EC_{50}$ values are reported in Table 6. The IL-2 moiety of all the constructs was successfully binding to the anti-IL-2 antibody (clone 5344) in ELISA and could be detected on the Fc-portion by an anti-human IgG antibody coupled to HRP (Table 5).

Binding to the CD122/CD132 complex (His-tagged, Acro Biosystem, Cat # ILG-H5283) of one representative anti-IL-2 antibody / IL-2 fusion (QTY065) was additionally assessed by SPR (BIAcore 3000, v4.1.2; GE Healthcare) and compared to Proleukin. CD122/CD132 was captured on an NTA sensor chip (GE Healthcare). Proleukin or QTY065 Fab were used as analytes. The calculated affinity constant for QTY065 Fab was 0.32 nM, comparable to the value of 0.42 nM obtained with Proleukin. The binding to the intermediate affinity IL-2R complex (CD122/CD132) remains unchanged in the IL-2CP antibody fusion proteins.

Table 5. Purity of the final protein by SEC-HPLC and assessment of functionality by ELISA and CD25 binding by SPR.

| Compound | Purity SEC-HPLC (%) | $EC_{50}$ to 5344 ELISA (nM) | KD to CD25 SPR (nM) |
|---|---|---|---|
| EAD406 | 97.61 | 0.11 | - |
| XFO227 | 97.12 | 0.14 | - |
| QTY065 | 99.10 | 0.14 | - |
| FJC828 | 97.77 | 0.28 | - |

(continued)

| Compound | Purity SEC-HPLC (%) | EC$_{50}$ to 5344 ELISA (nM) | KD to CD25 SPR (nM) |
|---|---|---|---|
| PGO345 | 95.75 | 0.16 | - |
| DRV470 | 96.85 | 0.10 | - |
| XUB802 | 94.58 | 0.15 | - |
| EPK959 | 99.20 | 0.15 | - |
| GYG794 | 97.80 | 0.20 | - |
| DRO069 | 97.52 | 0.11 | - |
| ECV200 | 96.80 | 0.13 | - |
| BFC885 | 97.74 | 0.25 | 54.1 |
| LPT269 | 97.47 | 0.24 | 0.12 |
| DXM339 | 97.93 | 0.28 | 0.18 |
| FUE433 | 98.61 | 0.54 | 0.16 |
| LQM346 | 99.09 | 0.28 | 0.15 |
| GLK754 | 98.61 | 0.28 | 0.20 |
| VBE401 | 92.55 | n.d. | - |
| LIZ707 | 91.55 | n.d. | - |

*Binding affinities to CD25 measured by surface plasmon resonance (SPR) analysis*

To provide optimal biased signaling to CD8 T cells, CD25 binding of IL-2 fusion proteins is preferably minimal. In order to understand if the CD25 binding site on the antibody IL-2 fusion proteins was accessible, binding to CD25 was assessed by surface plasmon resonance (SPR) analysis (BIAcore 3000, GE Healthcare, 33-1140587-3682). His-tagged recombinant CD25 was captured via TrisNTA Biotin on SA chips, and kinetic titration of IL-2 fusion proteins of the present invention was performed reaching concentrations up to 500 nM. IL-2 fusion proteins to Antibody A did not display binding to CD25 up to concentrations ≤ 500 nM. Antibody A binds IL-2 with high affinity on the CD25 binding site, thereby blocking IL-2 binding to the receptor CD25. Antibody B binds IL-2 with low affinity and allows IL-2 binding to CD25 only when fused to IL-2 with long linkers (≥13 and 14 amino acids). Antibody C has no affinity for IL-2 and allows CD25 binding of its fused IL-2 with any linker length (Table 4 and 5).

*pSTAT5 in mouse splenocytes (EC50 of Tregs, CD8, NK)*

STAT5 phosphorylation was analyzed in murine splenocytes as downstream signaling of the IL-2R activation. In order to assess the *in vitro* selectivity of the antibody-IL-2 fusion proteins, pSTAT5 was measured in different cell populations after stimulation with Proleukin, or with the compounds of the present invention. Freshly isolated murine splenocytes from C57BL/6 mice were incubated with a dilution series of Proleukin or IL-2-antibody fusion protein, starting from 100 nM. The cells were immediately fixed and stained with surface markers (i.e., CD25, CD3, NK1.1, CD4, CD8). After permeabilization (Perm III buffer, BD Biosciences), intracellular staining was performed (FoxP3, pSTAT5) before acquisition through Flow Cytometry. % of pSTAT5+ cells of CD8$^+$ T cells, NK cells and CD4$^+$CD25$^+$FoxP3$^+$ Tregs were plotted for each compound against molar concentration of IL-2 antibody fusion protein. EC50 values were calculated with GraphPad Prism (Table 6). Potency on Treg cells of IL-2 fusion proteins to Antibody A is markedly decreased compared to Proleukin, while the EC50 values on NK and CD8 T-cells are comparable. Fusion proteins to antibody B show reduced potency on Treg cells compared to Proleukin, although the biased effect gets reduced with increasing linker length (compound BFC885). IL-2 fused to Antibody C signals with high potency to Treg cells, comparable to Proleukin. Affinity of the antibody to the permuted IL-2 is required in order to efficiently provide steric hindrance and thereby exclude the CD25 from the signaling complex (Table 6).

Table 6. EC50 of STAT5 activation of mouse splenocytes of IL-2-antibody fusion proteins on NK cells, CD8 cells, and Treg cells.

| Compound name | EC$_{50}$ NK cells (nM) | EC$_{50}$ CD8 cells (nM) | EC$_{50}$ Treg cells (nM) |
|---|---|---|---|
| Proleukin | 1.4 | 4.1 | <0.016 |
| EAD406 | 5.2 | 15.7 | 22.0 |
| XFO227 | 2.7 | 8.0 | 10.6 |
| QTY065 | 1.2 | 4.6 | 5.91 |
| FJC828 | 1.9 | 5.8 | 8.4 |
| PGO345 | 2.3 | 6.6 | 7.6 |
| DRV470 | 2.4 | 7.9 | 10.3 |
| XUB802 | 5.8 | 17.1 | 12.1 |
| EPK959 | 1.9 | 16.0 | 16.0 |
| GYG794 | 1.5 | 18.6 | 4.8 |
| DRO069 | 0.9 | 6.8 | 6.6 |
| ECV200 | 0.9 | 6.9 | 3.7 |
| BFC885 | 2.0 | 26.4 | 0.9 |
| LPT269 | 1.7 | 32.0 | <0.016 |
| DXM339 | 1.3 | 18.6 | <0.016 |
| FUE433 | 1.2 | 15.7 | <0.016 |
| LQM346 | 1.8 | 25.4 | <0.016 |
| GLK754 | 2.5 | 70.5 | <0.016 |
| VBE401 | 34.3 | 32.2 | 2.6 |
| LIZ707 | 5.3 | 12.5 | 3.8 |

Applying the representative embedding procedures to IL-2-specific antibodies A or B resulted in fusion proteins with equally favorable IL-2 absence of CD25 binding (Table 5) and reduced potency in STAT5 phosphorylation on Treg cells compared to Proleukin (Table 6). This confirmed that by adjusting the orientation of the circularized IL-2 protein to conserve the natural binding orientation to an anti-IL-2 antibody to the cytokine, fusion to the heavy, or light chain variable region is feasible. A low affinity for IL-2 of antibody B (269 nM $K_D$) was sufficient to confer selective function of the resulting fusion protein. Peptide linkers of up to 20 amino acids in length retained desirable signaling qualities for the IL-2 fusion protein with Antibody A, though shorter peptides were preferred in combination with a lower affinity antibody. Fusion protein embedding IL-2CP in Antibody C did not exclude CD25 binding (even with 0-1 amino acid linkers), indicating that antibody affinity at least equivalent to Antibody B to IL-2 is required.

*Example 2: Generation of high affinity non-blocking anti-PD-1 antibodies*

*Identification of anti-hPD-1 antibodies, non-competing with PD-1 agonists*

Three anti-human PD-1 antibodies that were described as non-competing with PD-1 antagonists or non-PD-L1 blocking were identified (Table 7). These antibodies were tested for competition with commercial PD-1 antagonistic antibodies by flow cytometry using 20x molar excess of competitor. Raji PD-1 expressing cells (Invitrogen) were incubated 30 minutes at 4°C with serial dilutions of Pembrolizumab and Nivolumab, starting from 81 μg/ml (1:3 serial dilution). After washing the cells, antibodies in the first column of Table 8 labeled with biotin were added at a fixed concentration of 2 μg/ml. Bound antibodies were detected with Streptavidin-PE (Biolegend) and MFI levels of bound compound were compared to MFI in samples without competitor to determine the % signal inhibition. Background of samples incubated with Streptavidin-PE only was subtracted from all samples. Antibody XVT458 did not show any competition with any of the PD-1 antagonists tested. Antibody ZJN296 showed partial competition with Pembrolizumab, with a reduced mean fluorescence intensity (MFI) compared to sample with no competitor of 47.4%. Nonetheless, since the signal did not show a concentration dependent decrease, the reduced MFI in the sample with 20x competitor concentration may be unrelated to the presence of Pembrolizumab. Nivolumab did not significantly decrease the signal of antibody ZJN296. Full competition between antibody OVL714 was observed with both Pembrolizumab and Nivolumab, indicating a possible common epitope on the antigen.

Table 7. Antibodies described as non-competing with PD-1 antagonists or PD-L1 and source. Percent inhibition of binding signal to cells expressing human PD-1 when pre-coated with 20x molar excess of the indicated commercial PD-1 antagonist antibodies.

| Antibody | Species | Source | Pembrolizumab (% inhibition) | Nivolumab (% inhibition) |
|---|---|---|---|---|
| XVT458 | Humanized | (Clone NB01a) Fenwick C., J Exp Med, 2019 216 (7):1525 | 5.6 | 0 |
| ZJN296 | Mouse | (Clone SJL-565-4) Adler AS., mAbs, 2017 | 47.4 | 12.9 |
| OVL714 | Mouse | (Clone SJL-566-3) Adler AS., mAbs, 2017 | 99.5 | 99.3 |

*Re-humanization and affinity maturation of antibody XVT458*

The humanized VH sequence of XVT458, composed of shuffled frameworks from different families of germline genes, was re-humanized in order to obtain a VH with harmonic germline (GH), to provide z0-XVT458 (SEQ ID NOS 053, 054). Re-humanization of VH of XVT458 was performed by CDR grafting onto human IGHV1-18*01 IGHJ6*01 framework, which showed highest homology to the framework regions of the parental construct. Mutations of residues of the vernier zone were inserted in order to observe impact on affinity to the hPD-1 antigen. Germline (GH wild type or with point mutations) and parental humanized (uVH) were combined with the humanized VL (uVL) in a full hIgG1 format (Table 8).

Table 8. Derivatives of the anti PD-1 antibody XVT458 with newly humanized VH and binding kinetics measured by SPR.

| Analyte | Ligand | VH ' | VL | $k_a$ (1/Ms) | $k_d$ (1/s) | $K_D$ (M) |
|---|---|---|---|---|---|---|
| hPD-1 Avi-Tag Biotin | XVT458 | uVH | uVL | 8.30E+04 | 1.33E-03 | 1.60E-08 |
| | z0-XVT458 | GH | uVL | 9.11E+04 | 1.47E-03 | 1.61E-08 |
| | z1-XVT458 | GH+V067A | uVL | 9.23E+04 | 1.46E-03 | 1.59E-08 |
| | z2-XVT458 | GH+T071V | uVL | 9.07E+04 | 1.27E-03 | 1.40E-08 |
| | z3-XVT458 | GH+T073K | uVL | 8.74E+04 | 1.42E-03 | 1.62E-08 |
| | z4-XVT458 | GH+V067A+T071V | uVL | 9.88E+04 | 1.31E-03 | 1.33E-08 |
| | z5-XVT458 | GH+V067A+T073K | uVL | 9.14E+04 | 1.45E-03 | 1.59E-08 |
| | z6-XVT458 | GH+T071V+T073K | uVL | 9.20E+04 | 1.33E-03 | 1.45E-08 |
| | z7-XVT458 | GH+V067A+T071V+T073K | uVL | 9.57E+04 | 1.36E-03 | 1.43E-08 |
| | z8-XVT458 | GH+V067A+T071V+T073K | mVL | No or weak binding | | |

(continued)

| Analyte | Ligand | VH ' | VL | $k_a$ (1/Ms) | $k_d$ (1/s) | $K_D$ (M) |
|---|---|---|---|---|---|---|
| cynoPD-1 His | XVT458 | uVH | uVL | 5.57E+04 | 3.73E-03 | 6.70E-08 |
| | z0-XVT458 | GH | uVL | 6.32E+04 | 3.58E-03 | 5.67E-08 |
| | z1-XVT458 | GH+V067A | uVL | 6.27E+04 | 3.59E-03 | 5.73E-08 |
| | z2-XVT458 | GH+T071V | uVL | 5.72E+04 | 3.10E-03 | 5.42E-08 |
| | z3-XVT458 | GH+T073K | uVL | 5.74E+04 | 3.31E-03 | 5.77E-08 |
| | z4-XVT458 | GH+V067A+T071V | uVL | 6.33E+04 | 3.23E-03 | 5.10E-08 |
| | z5-XVT458 | GH+V067A+T073K | uVL | 5.86E+04 | 3.38E-03 | 5.73E-08 |
| | z6-XVT458 | GH+T071V+T073K | uVL | 5.84E+04 | 3.14E-03 | 5.38E-08 |
| | z7-XVT458 | GH+V067A+T071V+T073K | uVL | 5.89E+04 | 3.21E-03 | 5.44E-08 |
| | z8-XVT458 | GH+V067A+T071V+T073K | mVL | No or weak binding | | |

*Determination of $K_D$:*

The binding of each to PD-1 was assessed by SPR (Biacore 8K, GE Healthcare). Anti-human Fc IgG (Jackson) was immobilized on amine reactive CM5 chips (GE Healthcare) using 1xHBS-EP+ running buffer (GE Healthcare). The test antibodies were captured at flow rate 10 µl/min with 30 s contact time. Anti-PD-1 to human PD-1 (hPD-1) (AcroBiosystems, PD1-H82E4) and cynomolgus primate (cyno) PD-1 (cynoPD-1) (AcroBiosystems, PD-C5223) were run as analytes, diluted in 1xHBS-EP+ running buffer (GE Healthcare) with 240 s association and 600 s dissociation at flow rate 30 µl/min. Regeneration was carried out with 10 mM Glycine-HCl, pH 1.5 at flow rate 10 µL/min. This protocol is to be used, unless stated otherwise, in determination of Kd whereever used to define the $K_D$ of antibodies specified herein, with adaptions where necessary with regard to the nature of the interaction partners.

Binding of the antibody candidates on cell surface expressed antigen was confirmed by flow cytometry. CHO-S hPD-1 cells were incubated with a 5-fold serial dilution of the indicated humanized antibodies. Antibody binding was detected with goat anti-human IgG-PE. MFI were plotted over the antibody concentration to obtain EC50 values. Binding kinetics on SPR, and FACS EC50 binding values and maximal MFI values, indicated antibodies bound to hPD-1 expressing cells, except z8-uIgGKV326 (Tables 8 and 9).

Table 9. Top MFI values and EC50 values of anti-PD-1 antibodies binding to hPD-1 expressed on CHO-S cells.

| Ligand | Top MFI | FACS EC50 nM |
|---|---|---|
| XVT458 | 45100 | 8.60 |
| z0-XVT458 | 45185 | 5.74 |
| z1-XVT458 | 45235 | 5.85 |
| z2-XVT458 | 45528 | 4.15 |
| z3-XVT458 | 46192 | 5.47 |
| z4-XVT458 | 46726 | 4.54 |
| z5-XVT458 | 46173 | 4.51 |
| z6-XVT458 | 46791 | 3.93 |
| z7-XVT458 | 45991 | 3.88 |
| z8-XVT458 | 0 | NA |
| IgG1 isotype control | 0 | NA |

The humanized antibody z2-XVT458 (SEQ ID NOS 055, 056) was affinity matured by inserting random mutations in the CDRs (parsimonious mutagenesis method). Every position in the antibody VH and VL-CDRs was mutated by PCR using a mutagenic primer containing a degenerate codon NNS at a specific CDR position, introducing a combination of all 20 amino acids. The library of single mutants was screened by scFv capture ELISA using a redundancy factor of 4 (on 96-well plate er position). Clones displaying ≥ 2X ELISA signal compared to the wild-type scFv were sequenced, and clones with unique sequences were re-grown, re-screened by capture ELISA and ranked by dose-dependent ELISA on human and cyno antigens, and by FACS using human antigen expressing cells. Affinity improving mutations were used to design a combinatorial library using the Kunkel method. The library was screened by scFv capture ELISA using the redundancy factor of 4 (4x number of combinations). Clones displaying ≥ 2X ELISA signal compared to the wild-type scFv were sequenced, and clones with unique sequences were re-grown, re-screened by capture ELISA and ranked by dose-dependent ELISA on human and cyno antigens, and by FACS using human antigen expressing cells. Primary screening by saturated mutagenesis of antibody CDRs identified 25 mutants at 10 CDR amino acid positions that showed capture ELISA signal at least 2-fold higher than wild-type on hPD-1. 9 mutants showed improved binding to both recombinant hPD-1 and cynoPD-1, and hPD-1 on CHO-S cells. A combinatorial library was generated with these 9 mutants yielding 16 combinatorial variants with improved affinity to recombinant and cell-expressed antigen. From the base sequence of z2-XVT458 (variable domains SEQ ID NOs 055, 056), 6 subclones, z2-XVT458 m1-m6 (SEQ ID NOS 057 to 068), were selected for IgG conversion. In addition to the CDR mutations, the sequences of z2-XVT458-m1, z2-XVT458-m3 and z2-XVT458-m6 contain a G57D mutation just after the CDRL2, which may contribute to the increased affinity of these clones. The selected clones in IgG format showed a significant affinity improvement to recombinant hPD-1, cynoPD-1 and hPD-1 on cells. Compared to parental z2-XVT458, these clones (z2-XVT458m1-m6) also displayed 10-84 folds improvement in $k_{off}$ rate as measured by SPR on both hPD-1 and cynoPD-1 (Table 10; corresponding Seq ID NOs 057-068). For SPR the same method as above was used.

Table 10. Parental XVT458, z2-XVT458 and subclone affinity matured mutants in IgG format. Binding affinities measured by flow cytometry, ELISA and SPR.

| Abs | ELISA Binding | | | |
| | hPD-1 | | cynoPD-1 | |
| | EC50 (nM) | Max OD | EC50 nM(nM) | Max OD |
|---|---|---|---|---|
| XVT458 | 1.04 | 2.85 | 0.5 | 3.21 |
| XVT458-z2 | 0.71 | 2.91 | 0.4 | 3.32 |
| XVT458-z2-m1 | 0.083 | 3.39 | 0.087 | 3.58 |
| XVT458-z2-m3 | 0.089 | 3.37 | 0.094 | 3.54 |
| XVT458-z2-m5 | 0.051 | 3.42 | 0.056 | 3.58 |
| XVT458-z2-m6 | 0.064 | 3.49 | 0.071 | 3.69 |
| XVT458-z2-m4 | 0.079 | 3.3 | 0.098 | 3.54 |
| XVT458-z2-m2 | 0.076 | 3.34 | 0.086 | 3.56 |
| NC.hIgG1 | >50 | 0.05 | >50 | 0.07 |

| Abs | FACS Binding | | | |
| --- | --- | --- | --- | --- |
| | CHO-S hPD-1 | | CHO-S Cyno-PD-1 | |
| | EC$_{50}$ (nM) | Max MFI | EC$_{50}$ (nM) | Max MFI |
| XVT458 | 6.73 | 39900 | NA | 52 |
| XVT458-z2 | 5.86 | 40700 | NA | 109 |
| XVT458-z2-m1 | 1.5 | 41000 | NA | 70 |
| XVT458-z2-m3 | 1.34 | 41100 | NA | 124 |
| XVT458-z2-m5 | 0.84 | 42400 | NA | 335 |
| XVT458-z2-m6 | 1.56 | 39500 | NA | 59 |
| XVT458-z2-m4 | 0.96 | 43400 | NA | 258 |
| XVT458-z2-m2 | 1.15 | 42800 | NA | 367 |
| NC.hIgG1 | NA | 184 | NA | 206 |

| Abs | SPR | | | |
| --- | --- | --- | --- | --- |
| | hPD-1 | | | |
| | ka (1/Ms) | kd (1/s) | K$_D$ (M) | ka (1/Ms) |
| XVT458 | 9.48E+04 | 1.22E-03 | 1.28E-08 | 6.90E+04 |
| XVT458-z2 | 9.92E+04 | 1.24E-03 | 1.25E-08 | 7.30E+04 |
| XVT458-z2-m1 | 2.22E+05 | 3.28E-05 | 1.48E-10 | 1.85E+05 |
| XVT458-z2-m3 | 1.58E+05 | 6.57E-05 | 4.17E-10 | 1.38E+05 |
| XVT458-z2-m5 | 1.24E+05 | 9.77E-05 | 7.87E-10 | 1.11E+05 |
| XVT458-z2-m6 | 2.31E+05 | 1.01E-04 | 4.39E-10 | 2.13E+05 |
| XVT458-z2-m4 | 1.42E+05 | 1.73E-04 | 1.22E-09 | 1.34E+05 |
| XVT458-z2-m2 | 1.53E+05 | 1.90E-04 | 1.24E-09 | 1.27E+05 |
| NC.hIgG1 | / | / | / | / |

*Humanization of antibody ZJN296*

ZJN296 was humanized by CDR grafting using germlines with highest identity with the murine framework (IGKV1-33*01 IGKJ2 and IGHV1-18*01 IGHJ6*01). To increase the likelihood of retaining the binding affinity to the target antigen, 8 additional humanized VL-genes and 2 humanized VH-genes were designed by mutating human amino acids back to mouse. Such mutations may preserve the original conformation of VH and VL CDR loop and antigen binding if this loop makes contact with the antigen (Table 11).

Table 11. Antibody variants of humanized ZJN296, with mutations inserted in humanized ZJN296-0 VH (GH, SEQ ID NO 069) and VL (SEQ ID NO 070).

| ID | VH | VL | ID | VH | VL |
| --- | --- | --- | --- | --- | --- |
| ZJN296 | mVH | mVK | ZJN296-9 | GH+V002A | GL+S060D |
| ZJN296-0 | GH | GL | ZJN296-10 | GH+T071V | GL+S060D |

(continued)

| ID | VH | VL | ID | VH | VL |
|---|---|---|---|---|---|
| ZJN296-1 | GH+V002A | GL | ZJN296-11 | GH+T073R | GL+S060D |
| ZJN296-2 | GH+T071V | GL | ZJN296-12 | GH+V002A+T071V | GL+S060D |
| ZJN296-3 | GH+T073R | GL | ZJN296-13 | GH+V002A+T073R | GL+S060D |
| ZJN296-4 | GH+V002A +T071V | GL | ZJN296-14 | GH+T071V+T073R | GL+S060D |
| ZJN296-5 | GH+V002A +T073R | GL | ZJN296-15 | GH+V002A+T071V+ T073R | GL+S060D |
| ZJN296-6 | GH+T071V+ T073R | GL | ZJN296-16 | GH+V002A+V067A+ T071V+T073R | GL+S060D |
| ZJN296-7 | GH+V002A +T071V+T0 73R | GL | ZJN296-17 | mVH | GL+S060D |
| ZJN296-8 | GH | GL+S06 0D | ZJN296-18 | GH+V002A+V067A+ T071V+T073R | mVL |

Recombinant humanized antibody variants of ZJN296 were expressed as human IgG1 in HEK293 cells. For SPR analysis (Biacore 8K, GE Healthcare) anti-human Fc IgG (Bethyl, A80-304P) was immobilized on CM5 chips (Cytiva) using 1xHBS-EP+ running buffer. Humanized anti-PD-1 antibodies were captured on the chip. hPD-1 and cynoPD-1 were run as analytes 1xHBS-EP+ running buffer at a flow rate of 30 $\mu$L/min for an association phase of 180 s, followed by 400 s dissociation. 10 mM glycine (pH 1.5) as regeneration buffer was injected to flow cells following every dissociation phase.

Based on SPR binding kinetics on human and cyno antigen and binding data to hPD-1 expressed on cells tested with supernatants, 5 best candidates were expressed recombinantly and a full SPR kinetics was performed (Table 12). ZJN296-0 and ZJN296-6 were selected as candidates since ZJN296-0 has minimum back mutations and comparable binding potency compared with the chimeric antibody, and ZJN296-6 has the best binding characteristics.

Table 12. SPR kinetics results of humanized purified ZJN296 derived antibodies

| Sample Name | Binding to Human PD1 | | | Binding to Cyno PD1 | | |
|---|---|---|---|---|---|---|
| | $k_a$ (1/Ms) | $k_d$ (1/s) | $k_D$ (M) | $k_a$ (1/Ms) | $k_d$ (1/s) | $k_D$ (M) |
| ZJN296 | 7.71E+04 | 7.37E-04 | 9.56E-09 | 8.93E+04 | 1.15E-02 | 1.28E-07 |
| ZJN296-0 | 5.69E+04 | 4.95E-04 | 8.70E-09 | 6.15E+04 | 6.41E-03 | 1.04E-07 |
| ZJN296-2 | 5.65 E+04 | 3.24E-04 | 5.72E-09 | 5.96E+04 | 4.33E-03 | 7.27E-08 |
| ZJN296-3 | 5.61E+04 | 3.69E-04 | 6.57E-09 | 4.76E+04 | 5.11E-03 | 1.07E-07 |
| ZJN296-6 | 6.26E+04 | 2.01E-04 | 3.22E-09 | 6.36E+04 | 2.79E-03 | 4.39E-08 |

*Non-blocking antibody hybridoma screening*

High affinity binding antibodies specific for human PD-1 (hPD-1), cross-reactive with cynomolgus monkey PD-1 (cynoPD-1), but not blocking PD-L1 or PD-1 antagonist antibodies were generated by immunization of humanized mice (AlivaMab® mice) and hybridoma technology. Two separate immunization campaigns (with 10 Kappa-Lambda mice in first campaign, 4 Kappa and 4 Lambda in second campaign) were performed by immunization with a combination of h/cyPD-1. Primary functional screening was performed using hPD-1 and cyPD-1 transfected HEK293 cells. Binding was assessed by flow cytometry. 31 plates of 384 wells were screened in the first campaign, and 15 plates of 384 wells were screened in the second campaign. In the second screen selected clones were initially tested from supernatants for competition with Nivolumab and Pembrolizumab, testing binding on antigen expressing cells by flow cytometry in presence of the competitor antibodies and detecting with an anti-mouse IgG antibody. Non-competitors were determined as MFI(+ competitor)/MFI(no competitor) > 0.6. Only the non-competing clones, that showed high affinity binding on BLI (KD < 1 nM) were expanded and purified. Out of all the screened clones, 7 clones fitting the desired binding profile were identified (Table 13, SEQ ID NOs 071 to 084 VH and VL of antibodies that were used with $CH_{1,2,3}$ and corresponding CL(kappa) or CL(lambda)). Table 13 summarizes the binding properties to human and cyno PD-1. EC50 binding to HEK293 cells transfected with hPD-1 and cyPD-1 were measured by flow cytometry. Binding kinetics to the recombinant antigen was measured by BLI. Association (220s) and dissociation (480s) of a dilution series of recombinant hPD-1 and cynoPD-1 (100, 25, 6.25 nM, AcroBio) were measured on the antibody-coated biosensors (anti-humanIgG CH1 Biosensor, ForteBio) Octet-System (Octet RED, ForteBio). $K_D$ values were obtained by fitting the kinetics data with the ForteBio Data Analysis Software (8.2). PBS was used as assay buffer (PBS 10mM Phosphate, 150mM NaCl - pH 7.4). For dissociation the biosensors were dipped into kinetic assay buffer (PBS 10mM Phosphate, 150mM NaCl - 0.1% BSA - 0.02% Tween - pH 7.4). Sequencing of the final clones revealed that clones 21A08, 22F13 and 25I20 used the same HC/LC V-regions and have the same CDR3s. Clones 20H02, 39F23, 40B20 and 56H02 showed multiple developability liabilities in the CDR regions. Clone 21A08 was selected as the best clone for its affinity and purity from SEC-HPLC compared to the other sibling clones.

Table 13. FACS EC50 binding to hPD-1 or cynoPD-1 expressing HEK293 cells, SPR binding kinetics on hPD-1 and cynoPD-1.

|  | FACS EC50 (M) | | PD1-HIS | | | cynoPD-1-HIS | | |
| --- | --- | --- | --- | --- | --- | --- | --- | --- |
| clone | 293:huPD1 | 293:cyPD1 | KD (M) | kdis(1/s) | kon(1/Ms) | KD (M) | kdis(1/s) | kon(1/Ms) |
| 21A08 | 2.43E-10 | 1.95E-10 | 9.27E-12 | 6.39E-07 | 6.90E+04 | 1.24E-09 | 8.25E-05 | 6.67E+04 |
| 22F13 | 2.00E-10 | 1.07E-10 | 1.41E-09 | 1.32E-04 | 9.40E+04 | 8.45E-09 | 8.38E-04 | 9.92E+04 |
| 25I20 | 1.79E-10 | 3.10E-10 | 1.15E-09 | 1.01E-04 | 8.75E+04 | 8.38E-09 | 7.83E-04 | 9.34E+04 |
| 20H02 | 5.03E-10 | 2.31E-10 | 2.43E-09 | 2.21E-04 | 9.09E+04 | 6.39E-09 | 4.63E-04 | 7.25E+04 |
| 39F23 | 1.89E-11 | 2.89E-11 | 1.03E-09 | 2.53E-04 | 2.44E+05 | 1.30E-09 | 2.94E-04 | 2.27E+05 |
| 40B20 | 1.62E-10 | 9.58E-11 | 9.61E-09 | 7.73E-04 | 8.05E+04 | 7.51E-09 | 5.05E-04 | 6.73E+04 |
| 56H02 | 9.21E-11 | 8.81E-11 | 2.28E-10 | 3.79E-05 | 1.66E+05 | 2.62E-10 | 3.67E-05 | 1.40E+05 |

In order to remove an N-glycosylation site present in the CDR-L1, mutations were inserted in the CDR-L1 of 21A08. The first asparagine (Asp, N) in CDR-L1, was replaced by a serine (Ser, S), Glutamine (Gln, Q), or Alanine (Ala, A). Binding of the 21A08 clone variants (21A08S, 21A08Q and 21A08A) was tested by BLI and compared to the wt clone using supernatants of transiently transfected HEK293 cells, and a positive control (Pembrolizumab).

Table 14. Antibody clone 21A08 and deglycosylated variants with binding affinity (KD) and dissociation constant (Koff).

| Clone name | Mutations LCDR1 | KD huPD-1 (M) | Koff 1/s |
| --- | --- | --- | --- |
| 21A08 | - | 2.17 E-09 | 1.40 E-04 |
| 21A08S | N>S | 2.47 E-09 | 1.84 E-04 |
| 21A08Q | N>Q | 3.39 E-09 | 2.67 E-04 |
| 21A08A | N>A | 1.81 E-09 | 1.81 E-04 |
| Pembrolizumab |  | 6.29 E-09 | 1.61 E-03 |

In addition, a deamidation site present in the CDR-L1 was removed by inserting the mutations listed in Table 15. Binding kinetics of 21A08A and the deamidation site removal mutants were measured by SPR. For SPR analysis (Biacore 8K, GE Healthcare) anti-human Fc IgG (Bethyl, A80-304P) was immobilized on CM5 chips (Cytiva) and the test anti-PD-1 antibodies were captured using 1xHBS-EP+ running buffer at flow rate 10 μL/min. hPD-1 and cynoPD-1 (His -tagged) were run as analytes diluted in 1xHBS-EP+ running buffer at a flow rate of 30 μL/min for an association phase of 250 s, followed by 3600 s dissociation. 10 mM glycine (pH 1.5) as regeneration buffer was injected to flow cells following every dissociation phase. All clones lacking the N-glycosylation site retained binding to PD-1 (Table 14). None of the mutations in the CDR-L1 compromised the binding kinetics to hPD-1 or cynoPD-1 significantly.

Table 15. Antibody clone 21A08A and variants with deamidation site removed, binding affinity and dissociation constant

| Clone name | Mutation LCDR1 | ka hPD-1 (1/Ms) | kd hPD-1 (1/s) | KD hPD-1 (M) | ka cPD-1 (1/Ms) | kd cPD-1 (1/s) | KD cPD-1 (M) |
|---|---|---|---|---|---|---|---|
| 21A08A | - | 6.3E+04 | <1.0E-05 | <1.6E-10 | 5.6E+04 | 9.6E-05 | 1.7 E-09 |
| 21A08Ap1 | NS>QS | 5.7E+04 | 2.9E-05 | 5.0 E-10 | 5.2E+04 | 1.6E-04 | 3.0 E-09 |
| 21A08Ap2 | NS>SS | 6.4E+04 | 4.2E-05 | 6.6 E-10 | 5.9E+04 | 1.9E-04 | 3.2 E-09 |
| 21A08Ap3 | NS>NA | 6.0E+04 | 3.5E-05 | 5.8 E-10 | 5.5E+04 | 2.5E-04 | 4.67E-09 |

*Example 3: binding profile of non-blocking anti-PD-1 antibodies*

Assessment of the binding profile of non-blocking anti-PD-1 antibodies was then made using a human plasma membrane protein cell array. The Retrogenix Cell Microarray Technology platform screened the anti-PD-1 antibody candidates for cross-reactivity binding with non-target proteins. The test antibodies were each screened for binding against human HEK293 cells, individually expressing 6018 full-length human plasma membrane proteins, secreted and cell surface-tethered human secreted proteins plus a further 397 human heterodimers. In a pre-screen 2 $\mu$g/mL of each test antibody or PBS only was added to slides of fixed un-transfected HEK293 cells. Binding to un-transfected cells was assessed using an AlexaFluor647 labelled anti-human IgG Fc detection antibody (AF647 anti-hIgG Fc), followed by fluorescence imaging. In the pre-screen with antibody XVT458-z2-m5 a high background was detected. A second pre-screen was repeated with 0.5mg/ml, which resulted in background reduction. For library screening 6018 expression vectors, encoding both ZsGreen1 and a full-length human plasma membrane protein or a cell surface-tethered human secreted protein, were individually arrayed in duplicate across 17 microarray slides ('slide-sets'). In addition, vectors encoding a further 397 human heterodimers were co-arrayed across a further microarray slide. Human HEK293 cells were used for reverse transfection/expression. Test antibody XVT458-z2-m5 was added to each of the 18 slide-sets after cell fixation giving a final concentration of 0.5 $\mu$g/mL, the remaining 9 test antibodies were added at final concentration of 2 $\mu$g/mL. Detection of binding was performed by using the same fluorescent secondary antibody as used in the pre-screen (AF647 anti-hIgG Fc). Fluorescent images were analyzed and quantitated (for transfection) using ImageQuant software (GE healthcare, Version 8.2). A protein 'hit' is defined as a duplicate spot showing a raised signal compared to background levels. This is achieved by visual inspection using the images gridded on the ImageQuant software. Hits were classified as 'strong, medium, weak or very weak', depending on the intensity of the duplicate spots. The screen revealed 29 library hits. After removing 12 interactions which were observed with a test antibody and the control treatment and therefore designated as non-specific, a further 7 low confidence interactions, 10 specific interactions for the test antibodies were identified (Table 16). All test antibodies showed a single specific interaction with PD-1 (PDCD1). Test antibodies ZJN296-0, ZJN296-6, XVT458-z2-m1, XVT458-z2-m2, XVT458-z2-m4, XVT458-z2-m5 all showed medium to weak interactions with other target proteins. Clones 21A08Ap1 (SEQ ID NO VH 085, VL 086), 21A08Ap2 (SEQ ID NO VH 085, VL 087), XVT458-z2-m3 (SEQ ID NO 061, 062), and XVT458-z2-m6 (SEQ ID NO 067, 068) showed single specific interaction to the target of interest.

Table 16. Hits from the Retrogenix cell microarray screen. Protein types are Plasma membrane (PM), Secreted (S), Tethered Secreted (TS), Heterodimer (HD) or Evidence of plasma membrane (M). Hits no., above very weak intensity (next page)

| Sample Id and dose | Hits | Hit no. | 1 | 2 | 3 | 4 | 5 | 6 | 7 | 8 | 9 | 10 |
|---|---|---|---|---|---|---|---|---|---|---|---|---|---|
| | | Gene ID | PDCD1 | RTN4RL2 | PROM2 | APP | SV2A | DCC | RARRES2 | IGSF1 | CD248 | NLGN1 |
| | | Protein Type | PM | PM | PM | PM | PM | PM | TS | PM | PM | PM |
| | | Ref seq isoform (for | single form | Isoform 1 | Isoform 1 | Isoform APP751 | Isoform 1 | single form | single form | Isoform 1 | Isoform 1 | Isoform 2 |
| 21A08Ap1 (2 µg/mL) | 1 | | strong | | | | | | | | | |
| 21A08Ap2 (2 µg/mL) | 1 | | strong | | | | | | | | | |
| ZJN296-0 (2 µg/mL) | 2 | | strong | medium | | | | | | | | |
| ZJN296-6 (2 µg/mL) | 2 | | strong | medium | | | | | | | | |
| XVT458-z2-ml (2 µg/mL) | 2 | | strong | | medium | | | | | | | |
| XVT458-z2-m2 (2 µg/mL) | 6 | | strong | | medium | weak | weak | weak | weak | | | v.weak |
| XVT458-z2-m3 (2 µg/mL) | 1 | | strong | | | | | | | | | |
| XVT458-z2-m4 (2 µg/mL) | 4 | | strong | | | | | | | weak | weak | weak |
| XVT458-z2-m5 (0.5 µg/mL) | 2 | | strong | | | | | | weak | | | v.weak |
| XVT458-z2-m6 (2 µg/mL) | 1 | | strong | | | | | | | | | |
| PBS (secondary only)* | | | | | | | | | | | | |

EP 4 431 525 A1

*Example 4: Non-competitive binding of anti-PD-1 antibodies with PD-L 1 antagonists and PD-L 1*

*Competition on hPD-1 expressing cells*

Antibodies 21A08Ap1 and 21A08Ap2 were tested for competition with commercial PD-1 antagonistic antibodies by flow cytometry. Jurkat PD-1 expressing cells were incubated 30 minutes at 4°C with a serial dilution of Pembrolizumab and Nivolumab, starting from 10 $\mu$M (1:3 serial dilution). Without washing the cells antibodies 21A08Ap1 and 21A08Ap2 labeled with biotin were added at a fixed concentration of 100 nM. Bound antibodies were detected with Streptavidin-PE (Biolegend) and MFI levels of bound compound were compared to MFI in samples without competitor to determine the % signal inhibition. Background of samples incubated with Streptavidin-PE only was subtracted from all samples. Inhibition was minimal, under 20%, showing neither Pembrolizumab nor Nivolumab significantly decreased the signal of antibodies 21A08Ap1 and 21A08Ap2 (Table 17).

Table 17. Competition on hPD-1 expressed on cells with commercial PD-1 antagonists, Pembrolizumab and Nivolumab. Flow cytometry % signal inhibition of the tested antibody using 100x molar excess of competitor.

| Antibody name | Signal inhibition in % with 100x molar excess | |
|---|---|---|
| | Pembrolizumab | Nivolumab |
| 21A08Ap1 | 11.1 | 7.3 |
| 21A08Ap2 | 4.4 | 0 |

*Simultaneous binding of hPD-1 to 21A08Ap1 and PD-L1*

Simultaneous binding of PD-1 (Fc-Tag) to anti-PD-1 antibodies and PD-L1 (His-Tagged, Acrobiosystems, #H52H3) was assessed by SPR analysis using the Biacore T200 (Cytiva, #28975001). PD-L1 was captured on an anti-His-CM5 chip (Cytiva, chip: #29104988, His Capture Kit: #28995056). 1 $\mu$M of PD-1 was premixed with 5 $\mu$M of antibody (Pembrolizumab, IgG1 isotype control antibody, or 21A08p1) or running buffer (Xantec, HBSTE: B HBSTE10) for at least 30 min before binding analysis (association and dissociation 60s each). Data was analyzed using the Biacore Insight Evaluation Software (Cytiva, V4.0.8 # 29310606). Double reference subtraction was performed (surface without ligand and running buffer injection). Figure 3 reports the Biacore sensograms. No binding (as change in RU) was observed for PD-1 pre-mixed with Pembrolizumab, confirming that Pembrolizumab blocks the PD-L1 binding site of PD-1. By contrast, the complex of PD-1 with the antibody 21A08Ap1 was still able to bind to PD-L1, giving a signal of approximately 60 RU. The controls of PD-1 alone and PD-1+IgG1 isotype control showed an association signal of approximately 30 RU.

*Example 5: Rational design of non-blocking PD-1 targeted IL-2 fusion proteins*

Proof-of-concept compounds were designed and characterized to determine the most favorable format for adding a non-blocking PD-1 targeting moiety to a CD122/CD132 dimeric receptor biased IL-2-anti-IL-2 antibody fusion moiety. The anti-PD-1 antibodies XVT458 or ZJN296 (Table 7) were combined with Antibody A - IL-2 fusion protein (QTY065, SEQ ID NO 051, 052) to generate bispecific compounds delivering specifically CD122-CD132 biased IL-2 to PD-1 expressing cells. Five formats were designed different in size and valency to the PD-1 antigen (Fig. 4, Table 18). The double-scFv fusion comprises the anti-PD-1 antibody XVT458 in scFv format, i.e. the VH and VL domains fused by a 15 amino acid Glycine(G)-Serine(S) linker, fused to the QTY065 IL-2-antibody fusion scFv by a G4S linker (Fig. 4A). The Fab-double scFv consists of the QTY065 antibody-IL-2 fusion as Fab fragment (IL-2-VL-CL and VH-CH1) fused at each C-terminus to two anti-PD-1 scFv fragments of the XVT458 antibody (by their N-terminus VH-$(G_4S)_3$-VL, Fig. 4B). The IgG CrossMab format is a heterotetrameric human IgG1, with HC1 and LC1 from the anti-PD-1 antibodies XVT458 or ZJN296 and HC2 and LC2 from antibody-IL-2 fusion QTY065 (Fig. 4C). Knob into hole mutations (Y407T on HC1 and T366Y on HC2) were used to ensure correct heavy chain pairing. To enhance correct light chain pairing the CL and CH1 domains of HC1 and LC1 were swapped (WO2009080253A1). For Fc silencing of the IgG1 Fc region the mutations L234A, L235A, P329G (WO2012130831A1) were inserted on both HC1 and HC2. The IgG-scFv fusion proteins include the antibody XVT458 as full human IgG1 (consisting of HC1, identical LCs, HC2) having the HC2 fused on either its N-terminus (Fig. 4E) or C-terminus (Fig. 4D) to the IL-2-antibody fusion QTY065 as scFv (i.e., IL-2-VL$_{AntibodyA}$-$(G_4S)_3$ - VH$_{AntibodyA}$- $(G_4S)_2$ - HC2$_{XVT458}$ or HC2$_{XVT458}$- $(G_4S)_{2/4}$-IL-2-VL$_{AntibodyA}$-$(G_4S)_3$ - VH$_{AntibodyA}$). Pairing to HC1 is enhanced by the knob into hole mutations (same as CrossMab) and Fc silencing by L234A, L235A, P329G mutations. The bispecific antibodies were produced in Expi297 cells as described for IL-2 fusion proteins described above.

The functionality of the bispecific antibody was assessed with a sandwich ELISA that relies on its binding to the target antigen (coated hPD-1) and the integrity of the fused IL-2 via a secondary antibody (anti-IL-2 clone 5344). 60 nM of hPD-1 (ECD-His, produced in house) was coated on a Maxisorp plate (Nunc) overnight at 4°C and blocked with 5% BSA in PBS. Bispecific antibodies were added in a serial dilution in assay buffer and detected with biotinylated-5344 and Streptavidin HRP (BD Pharmingen, 554066). Absorbance signal after adding TMB was read with a plate reader (Spectramax ID3) at 450 nm. EC50 values were determined by blotting absorbance against concentration (Graphpad Prism, sigmoidal curve fit, 4 PL, X-axis is log). All compounds showed binding on both bispecific moieties; formats with bivalent binding to hPD-1 showed lower EC50 values compared to the monovalent formats (Table 18). Binding to hPD-1 expressed on the surface of Jurkat-PD-1 cells was confirmed by flow cytometry (Table 18). All formats retained binding to hPD-1. As expected, compounds bearing two PD-1-binding domains showed increased binding to hPD-1 due to avidity effects.

Table 18. Anti-PD-1, anti-hIL-2-IL-2 bispecific antibodies in various formats, with valency, molecular weight (Mw), EC50 to hPD-1 measured by ELISA and binding to hPD-1 expressed on Jurkat-hPD-1 cells measured by flow cytometry (MFI fold over background).

| Compound ID | Anti PD-1 antibody | Format | Binding arms to hPD1 | Anti-hIL-2/ IL-2 arms | Mw (kDa) | EC50 (nM) | hPD-1 binding (fold) |
|---|---|---|---|---|---|---|---|
| HRL470 | XVT458 | Double scFv fusion | 1 | 1 | 67.7 | 1.24 | 41.9 |
| VNP090 | XVT458 | Fab- double scFv | 2 | 1 | 116 | 0.11 | 19.9 |
| FQQ111 | XVT458 | IgG format with CrossMab$^{CH1-CL}$ | 1 | 1 | 161 | 1.16 | 46.5 |
| ONG682 | ZJN296 | IgG format with CrossMab$^{CH1-CL}$ | 1 | 1 | 161 | 0.87 | 20.1 |
| YMI345 | XVT458 | IgG with N-terminal scFv | 2 | 1 | 187 | 0.12 | 54.8 |
| CUM013 | XVT458 | IgG with C-terminal scFv, (G4S)4 linker | 2 | 1 | 188 | 0.11 | 47.3 |
| QWT744 | XVT458 | IgG with C-terminal scFv, (G4S)2 linker | 2 | 1 | 187 | 0.12 | 42.7 |

## Example 6: In vitro and in vivo cellular selectivity

In order to assess the functionality of the anti-IL-2/IL-2 fusion protein arm of the bispecific compounds, STAT5 phosphorylation was analyzed in murine splenocytes as downstream signaling of the IL-2R activation. pSTAT5 was measured in different cell populations after stimulation with Proleukin, or with one of the bispecific compounds. Mouse splenocytes were incubated with a serial dilution of bispecific compounds diluted in RPMI + 10% FBS. Starting concentration 100 nM, dilution rate 1:5 obtaining 6 total concentrations. Samples were incubated at 37 °C for 15 minutes and fixed immediately afterwards by adding same volume of cytofix buffer (BD Biosciences, Cat#554655) per sample and incubating samples for 10 minutes at 37 °C. After fixation cells were stained with BV421 rat anti-mouse CD25 (clone PC61, BD Biosciences, 0.5 $\mu$L/samples), BV650 hamster anti-mouse CD3e (clone 145-2C11, BD Biosciences, 1 $\mu$L/samples), BV711 mouse anti-mouse NK1.1 (clone PC136, BD Biosciences, 0.33 $\mu$L/samples) for 30 minutes at room temperature. Subsequently, cells were permeabilized using Perm Buffer III (BD Biosciences, Cat#558050) incubating for 10 minutes on ice. A second staining included PE-CF594 rat anti-mouse CD4 (clone RM4-5, BD Biosciences, 0.25 $\mu$L/samples), APC-780 rat anti-mouse CD8b (clone H35-17.2, eBioscience, 0.167 $\mu$L/samples), AF488 rat anti-mouse FoxP3 (clone FJK-16, eBioscience, 0.5 $\mu$L/samples), AF647 mouse anti-mouse pSTAT5 (clone pY694, BD Bioscience, 20 $\mu$L/samples). All samples were acquired with the software SpectroFlo® on the Cytek® Aurora. The .fcs files were analyzed with FlowJo_v10.6.2. EC50 of %pSTAT5+ values were obtained by sigmoidal 4-PL fitting. % of pSTAT5+ cells were plotted for each cell population and each tested compound against the log concentration (M).

The obtained EC50 values are shown in Table 19. All formats, except the IgG C-terminal scFv bispecifics, showed comparable (or max. 10 fold lower) potency in inducing STAT5 phosphorylation in NK and CD8 T cells compared to Proleukin (Table 19). EC50 values for QWT744 and CUM013 could not be determined, as the %pSTAT5 positive cells with the highest concentration of compound tested (100 nM) did not reach maximal levels. All compounds showed reduced potency in inducing pSTAT5 in Treg cells compared to Proleukin, indicating that signaling through the high affinity trimeric IL-2R is hindered *in vitro* (Table 19).

Table 19 Bispecific compounds, QTY065, and Proleukin EC50 values for pSTAT5 positive cells in parental NK, CD8, and Treg cells measured by flow cytometry in mouse splenocytes.

| Compound ID | EC50 pSTAT5 on NK cells (nM) | EC50 pSTAT5 on CD8 cells (nM) | EC50 pSTAT5 on Treg cells (nM) |
|---|---|---|---|
| Proleukin | 2.51 | 11.92 | < 0.02 |
| QTY065 | 2.88 | 17.93 | 4.21 |
| HRL470 | 4.04 | 27.45 | 1.18 |
| VNP090 | 14.67 | 26.81 | 10.94 |
| FQQ111 | 27.34 | 34.1 | 6.55 |
| YMI345 | 23.77 | 29.6 | 13.67 |
| CUM013 | > 100.00 | > 100.00 | > 100.00 |
| QWT744 | > 100.00 | > 100.00 | > 100.00 |
| ONG682 | 14.08 | 23.87 | 12.72 |

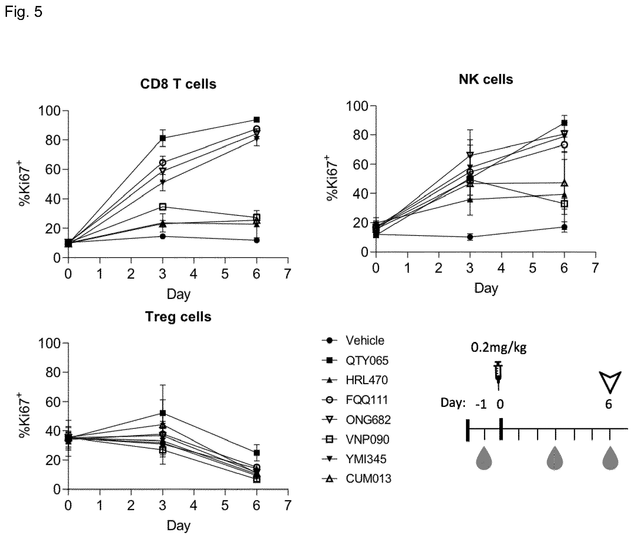

To confirm selectivity towards CD122-CD132 expressing cells *in vivo*, bispecific compounds were administered i.v. at 0.2mg/kg to wt C57BL/6 mice. Blood samples were taken before the injection and on days 3 and 6 after compound administration. Cell proliferation was analyzed by flow cytometry, detecting Ki67+ cells in CD8 T cells, NK cells and Treg cells (Fig. 5). C57BL/6 mice were injected i.v. with a single dose of 0.2 mg/kg compound. Blood samples taken before compound administration and on days 3 and 6 after compound administration were processed and analyzed by flow cytometry. Cells were first stained for CD25, CD3, NK1.1, CD4, CD8 and Zombie aqua fixable viability dye (Biolegend). After fixation and permeabilization (eBioscience) intracellular staining was performed for Ki67 and FoxP3. All compounds showed only slight increase in Ki67 expression in Treg cells on day 3, with levels decreasing on day 6 after compound administration. Proliferation (Ki67 expression) of CD8 T cells and NK cells in the blood of animals treated with bispecific compounds in the IgG format (FQQ111 and ONG682) or IgG-N-ter scFv (YIM345) format was comparable to QTY065, reaching maximal levels of Ki67+ cells (>75%) on day 6 after administration. Compounds with smaller molecular weight (Mw) and lacking the Fc domain, as HRL470 and VNP090, showed Ki67+ levels of 22-25% in CD8 T cells and 33-40% in NK cells. The bispecific format with C-terminal fusion of anti-hIL-2/IL-2 scFv on the anti-PD-1 IgG (CUM013) showed reduced potency compared to the other Fc-domain bearing compounds, with 47% Ki67+ NK cell and 25% Ki67+ CD8T cells, suggesting surprisingly, the IL-2 signaling was less potent when the cytokine was in a C-terminal linkage to a targeting antibody domain (Fig. 5).

*Example 7: In vivo anti-tumor efficacy*

The B16F10 melanoma model, an aggressive, and immune checkpoint inhibitor (CPi) resistant tumor model, was selected to investigate additional protection conferred by non-blocking, PD-1-targeted moieties compared to anti-IL-2/IL-2 fusion proteins lacking PD-1 targeting. Immune cell proliferation and immunophenotyping of B16F10 subcutaneous tumors was performed in humanized PD-1 mice so that both PD-1 targeting and IL-2 activity could be assessed (C57BL/6N-Pdcd1tm1(huPDCD1-ICP11; Geno). Transgenic hPD-1 mice were injected s.c. in the right flank with $1 \times 10^6$ B16F10 cells. When tumors reached an average size of 70 to 100 mm$^3$, compounds were administered i.v. at a dose of 0.2 mg/kg. Tumor volumes were measured daily with a caliper and calculated with the formula (length $\times$ width $\times$ width) 12. Tumor volume differences in % compared to vehicle at study end at day 6 after treatment onset were calculated. Tumor infiltrating cells were analyzed by flow cytometry as described above, using the antibodies shown in Table 20.

Table 20: Antibodies used for intracellular (IC) and extracellular staining (surface) for immunophenotyping of B16F10 tumors.

|  | Antigen | Label | Clone | Supplier | Catalog No |
|---|---|---|---|---|---|
| Surface | CD45 | BV480 | 30-F11 | BD Biosciences | 566095 |
|  | CD11b | BV605 | M1/70 | BD Biosciences | 563015 |
|  | F4/80 | BV605 | T45-2342 | BD Biosciences | 743281 |
|  | CD3 | BV650 | 145-2C11 | BD Biosciences | 564378 |
|  | CD4 | PE-CF594 | RM4-5 | BD Biosciences | 562285 |
|  | CD8 | R718 | H35-17.2 | BD Biosciences | 752290 |
|  | CD25 | BB515 | PC61 | BD Biosciences | 564424 |
|  | CD19 | SparkBlue | 6D5 | Biolegend | 115566 |
|  | hPD1 | PE | A17188B | Biolegend | 621607 |
|  | CX3CR1 | BV785 | SA011F11 | Biolegend | 149029 |
|  | NK1.1 | BV711 | PK136 | BD Biosciences | 740663 |
|  | CD49b | BB700 | HM$\alpha$2 | BD Biosciences | 742140 |
| IC | FoxP3 | BV421 | MF23 | BD Biosciences | 562996 |
|  | TCF1 | AF647 | C63D9 | Cell Signaling | 6709 |
|  | Ki67 | PE-Cy7 | RMT3-23 | Biolegend | 119716 |

While administration of the untargeted anti-IL-2/IL-2 fusion protein QTY065 mainly led to increased intratumoral NK cells in the tumor of treated mice (8.2 fold compared to vehicle), all PD-1 targeted compounds drove a marked increase of CD8 T cells in B16F10 tumors. The smaller format HRL470 with double scFv fusion has the smallest effect (2.7 fold increase compared to vehicle), while the IgG and IgG-scFv fusion lead to CD8 T cell increase 4.6-8.1 fold compared to vehicle. Notably, the effect was enhanced within the target cell population, CD8+PD-1+ cells (Table 21). The C-terminal scFv compound CUM013, which did not show high potency in previous assays, showed comparable effects to the other bispecific formats on tumor infiltrating lymphocytes (TILs). Nonetheless, CUM013 had smaller effect on tumor growth inhibition compared to the other bispecifics (Table 22). Based on the results obtained, heterotetrameric IgG (CrossMab format) and IgG-N'terminal-scFv-IL-2-fusion format bispecific molecules were particularly effective *in vivo,* and the formats of VNP090 and CUM013 were not pursued further and shelved as potential backup.

Table 21: TILs from transgenic hPD-1 mice treated with untargeted or PD-1 targeted IL-2 antibody fusion protein as fold increase cells/gram tumor over vehicle. Tumors analyzed on day 6 (n=4).

|  | Fold over vehicle cells/gram tumor | | | |
|---|---|---|---|---|
| Compound ID | Treg cells | NK cells | CD8 T cells | CD8 PD-1+ |
| QTY065 | 0.82 | 8.21 | 1.73 | 0.38 |
| HRL470 | 1.81 | 0.88 | 2.75 | 3.23 |
| VNP090 | 1.51 | 1.84 | 5.26 | 5.20 |
| FQQ111 | 1.25 | 1.12 | 4.62 | 5.52 |
| YMI345 | 0.97 | 1.78 | 5.32 | 6.36 |
| CUM013 | 0.83 | 0.80 | 8.14 | 9.83 |

Table 22: Tumor volume difference in % compared to mice treated with vehicle on day 6, n=4 mice per group.

| Compound ID | Tumor growth inhibition compared to vehicle (day 6) |
|---|---|
| QTY065 | -11.1% |

(continued)

| Compound ID | Tumor growth inhibition compared to vehicle (day 6) |
|---|---|
| HRL470 | 39.8% |
| VNP090 | 4.1% |
| FQQ111 | 46.6% |
| YMI345 | 33.0% |
| CUM013 | 8.7% |

In an additional study, tumor growth in hPD-1 transgenic mice was monitored after two administrations of bispecific compounds in comparison to vehicle, a non-blocking PD-1 antibody lacking IL-2 binding (the antibody XVT458 as human IgG1, with Fc silencing), or the untargeted bivalent antibody-IL-2 fusion QTY065. Day 0 corresponds to study start, when tumors reached 70-100mm$^3$. On day 0, and 3, 1.25 nmoles / kg of each compound were administered i.v. tumor growth was monitored and tumor volume difference was compared to mice treated with vehicle at end of study (Table 23). On day 5 after treatment, tumors were processed and TILs were analyzed by flow cytometry with intracellular staining as shown in Table 20. The inventors determined the fold increase compared to vehicle of cells/gram tumor of Treg cells, NK cells, CD8 T cells and CD8 T-cells subfamilies including stem-like pre-exhausted CD8 T cells (CD8+PD-1+TCF1+), better effector exhausted T cells (CD8+TCF1-CX3CR1+) and terminally exhausted T cells (CD8+TCF1-CX3CR1-). The control antibody XVT458 induced only a minimal increase of the tumor cell infiltrate. The bispecific compounds induced in particular increase of CD8+PD-1+ T cells and derived subfamilies, with the heterotetrameric IgG CrossMab and IgG-scFv N-terminal format being the most efficacious compounds, with regards to target cell targeting, and anti-tumor potency (Tables 23 and 24).

Table 23: Tumor volume difference in % compared to mice treated with vehicle on d5 (n=5-6).

| Compound ID | Tumor growth inhibition % |
|---|---|
| QTY065 | 34.6% |
| HRL470 | 41.0% |
| FQQ111 | 70.5% |
| ONG682 | 59.3% |
| YMI345 | 54.5% |
| XVT458 | 17.4% |

Table 24: TILs from transgenic hPD-1 mice treated with untargeted or PD-1 targeted IL-2 antibody fusion protein as fold increase cells/gram tumor over vehicle. Tumors analyzed on day 5 post initiation of treatment.

| Compound ID | Fold over vehicle cells/gram tumor | | | | | | |
|---|---|---|---|---|---|---|---|
| | Treg cells | NK cells | CD8 T cells | CD8 PD-1 + | CD8+PD-1+ stem-like | CD8+PD-1+ better effector | CD8+PD-1+ TCF1-exhausted |
| QTY065 | 1.7 | 63.0 | 4.9 | 3.5 | 13.1 | 3.4 | 5.3 |
| HRL470 | 2.2 | 3.6 | 3.1 | 3.0 | 3.9 | 2.8 | 4.5 |
| FQQ111 | 5.3 | 15.4 | 30.7 | 31.9 | 33.1 | 21.7 | 35.2 |
| ONG682 | 5.1 | 9.4 | 28.4 | 30.1 | 20.6 | 20.7 | 32.8 |
| YMI345 | 4.1 | 14.6 | 22.1 | 48.4 | 39.0 | 30.3 | 43.3 |
| XVT458 | 2.4 | 2.6 | 3.5 | 3.6 | 1.7 | 3.1 | 4.0 |

*Example 8: Increased in vitro potency of PD-1 targeted anti-hIL-2/IL-2 fusion proteins*

Select bispecific formats were then combined with well-performing non-blocking anti-hPD-1 antibodies (Table 25). Antibodies targeting irrelevant antigens MDC982 and KVC110 were used as untargeted control antibodies. The anti-hIL-2/IL-2 arm from QTY065 has a light chain from the kappa subfamily, and can be combined with a second arm being from the lambda subfamily to form a heterotetrameric compound (a bispecific antibody where each antigen binding domain has a different specificity), without need for genetic engineering strategies such as CrossMab to ensure correct light chain pairing. Bispecifics in the heterotetrameric IgG kappa/lambda (Figure 4F) format were designed combining the IL-2-anti-IL-2 fusion portion of QTY065 with two representative non-blocking, high affinity PD-1 antibodies 21A08Ap1 and 21A08Ap2 (both having a lambda light chain). Constructs encoding the sequences disclosed in Table 25 were transfected into Expi293 cells and produced and purified as described above. Knob in hole mutations were Y407T on HC1 and T366Y on HC2, and Fc silencing mutations L234A, L235A, P329A were incorporated.

Table 25: Combinations of QTY065 VH, VL(IL-2), and a second anti-hPD1 (or control) antibody heterodimer in 3 different bispecific antibody formats.

| Anti-PD-1 Antibody (light chain) | Bispecific antibody formats combined to QTY065 (kappa) (SEQ ID NO of polypeptide components) | | |
| --- | --- | --- | --- |
| | IgG CrossMab | IgG-scFv N-terminal | IgG kappa/lambda |
| 21A08A p1 (lambda) | YPW986 (088, 089, 090, 052) | LTJ498 (094, 095, 096) | XWY176 (094, 095, 100, 052) |
| 21A08A p2 (lambda) | PXU588 (092, 093, 090, 052) | JLI141 (097, 098, 099) | GQM289 (097, 098, 100,052) |
| XVT458-z2-m3 (kappa) | RIB426 (101, 102,090,052) | TMU471 (105, 106, 107) | - |
| XVT458-z2-m6 (kappa) | QAB373 (103, 104, 090, 052) | MDS446 (108, 109, 110) | - |
| Control Antibody (light chain) | IgG CrossMab | IgG-scFv N-terminal | IgG kappa/lambda |
| KVC110 (kappa) | TSQ225 | LNX431 | - |
| MDC982 (lambda) | - | - | UVW948 |

The bispecific compounds were tested for potency in inducing STAT5 phosphorylation in PD-1+ Jurkat cells, expressing the IL-2R CD122-CD132. Jurkat-PD1+ CD122+ cells were activated with a serial dilution of bispecific compounds for 15 minutes at 37°C and fixed by adding an equal volume of Cytofix buffer (BD Biosciences) for 10 minutes at 37°C. For the staining of intracellular antigens, cells were permeabilized with ice cold Perm buffer III (BD Biosciences) for 15 minutes on ice. Phosphorylated STAT5 was stained using an anti-pSTAT5 pY694 antibody (clone 47/Stat5, BD Biosciences). Flow cytometry was performed as previously described. EC50 values were calculated using the formula $Y=Bottom + (X^{Hillslope})*(Top-Bottom)/(X^{HillSlope} + EC50^{HillSlope})$ in Graphpad Prism V9.3.1 ([Agonist] vs. response - Variable slope (four parameters)). The PD-1 targeted bispecific compounds, except YPW986 and PXU588, showed increased potency and lower EC50 values compared to the bivalent QTY065 and the untargeted compound TSQ225 (Table 26). The binding ELISA assay suggested YPW986 and PXU588 (i.e. heterotetrameric IgG antibodies in a CrossMab bispecific format, with one arm derived from QTY065 and one either from 2108Ap1 or 21A08Ap2) lost their binding affinity to PD-1, in contrast to the heterotetrameric kappa/lambda IgG formats using the same non-blocking anti-PD-1 clones XWY176 and GQM289, which retained their ability to stimulate the dimeric IL-2 receptor. In general, PD-1 anchoring to the cells increased the signaling potency of the IL-2 on the bispecific compounds. The heterotetrameric Crossmab bispecific IgG FQQ111 with non-affinity matured anti-PD-1 arm (XVT458) induced a lower increase in potency by targeting to PD-1, indicating that higher affinity binding is desirable.

Table 26: EC50 values for STATS phosphorylation on Jurkat-PD-1+ CD122+ cells.

| Compound | EC50 value for pSTAT5 induction (nM) |
| --- | --- |
| MDS446 | 0.06 |
| TMU471 | 0.06 |
| QAB373 | 0.07 |
| RIB426 | 0.12 |
| JLI141 | 0.20 |

(continued)

| Compound | EC50 value for pSTAT5 induction (nM) |
|----------|--------------------------------------|
| GQM289 | 0.22 |
| LTJ498 | 0.24 |
| XWY176 | 0.27 |
| FQQ111 | 0.62 |
| QTY065 | 1.07 |
| TSQ225 | 5.16 |
| PXU588 | 5.37 |
| YPW986 | 43.9 |

A further functional effect of a fusion protein according to the invention, is decrease of cell surface PD-1 upon binding to the IL-2R. Signaling through the IL-2R leads to internalization of the dimeric or trimeric receptor complex (Robb RJ. Et al. J Exp. Med. (1987) doi: 10.1084/jem.165.4.1201). Reduction of cell surface hPD-1 and CD122 by immunoconjugates of the present invention was tested by incubating the bispecifics at 3 different concentrations with stimulated PBMCs (i.e., PD-1+) for 16 hours. As controls, the bivalent anti-hIL-2/IL-2 compound QTY065 and the untargeted IL-2 compound as IgG CrossMab were used. Buffy coats or whole blood from healthy volunteers were received from the Blutspendezentrum SRK beider Basel or Aarau in compliance with the Swiss Human Research Act (HRA; May 2021) and other applicable ethics regulations by the Swiss ethics committee. PBMCs were isolated by density gradient centrifugation using Ficoll Plaque Plus (GE Healthcare). Frozen PBMCs were defrosted activated for 3 days with plate coated anti-CD3 (clone OKT3, BioLegend) and soluble anti CD28 (clone CD28.2, BioLegend). After activation PBMCs were incubated with bispecific compounds as indicated, overnight at 37°C. After incubation, cells were fixed immediately by adding an equal volume of Cytofix buffer (BD Biosciences) for 10 minutes at 37°C. Subsequently, surface markers were stained using antibodies listed in Table 27. Cells were acquired by flow cytometry as described above.

Table 27: Antibodies used for extracellular staining for cell surface expression experiments.

| Antigen | Label | Clone | Supplier | Catalog No |
|---------|-------|-------|----------|------------|
| CD4 | PE-CF594 | SK3 | BD Biosciences | 566914 |
| CD8 | BV605 | SK1 | BD Biosciences | 564116 |
| CD122 | PE Cy7 | CF1 | BeckmanCoulter | A53365 |
| PD-1 | BB700 | EH12.1 | BD Biosciences | 746185 |
| CD3 | BV786 | SK7 | BD Biosciences | 563800 |
| CD132 | APC | TUGh4 | Biolegend | 338608 |
| CD25 | BV421 | 2A3 | BD Biosciences | 564033 |
| CD4 | PE-CF594 | SK3 | BD Biosciences | 566914 |

The difference in MFI of detected surface CD122 and PD-1 was calculated as % decrease compared to PBMC that were incubated only in medium. The control compounds induce decrease surface CD122, but leave PD-1 levels unchanged. For the PD-1 targeting compounds, both CD122 and PD-1 levels decreased. This may indicate that binding to the IL-2R is required for reduction of cell surface PD-1 (Table 28).

Table 28: Percentage decrease of CD122 and PD-1 MFI on CD8 T cells after incubation with control compounds or bispecific compounds compared to CD8 T cells incubated with medium alone (n= 2 donors).

| | % MFI decrease | | | | | |
| --- | --- | --- | --- | --- | --- | --- |
| | CD122 | | | PD-1 | | |
| Compound | 25nM | 5nM | 1 nM | 25nM | 5nM | 1nM |
| QTY065 control | 76.6 | 67.0 | 41.6 | - | - | - |
| TSQ225 control | 78.0 | 61.7 | 40.7 | - | - | - |
| LTJ498 | 59.1 | 36.2 | 7.8 | 46.4 | 46.7 | 41.8 |
| XWY176 | 62.9 | 40.1 | 9.4 | 52.4 | 52.7 | 42.0 |
| JLI141 | 60.7 | 35.3 | 5.7 | 38.6 | 45.0 | 38.0 |
| GQM289 | 57.4 | 39.9 | 9.7 | 46.4 | 44.8 | 25.7 |
| TMU471 | 59.8 | 43.4 | 24.7 | 34.6 | 46.4 | 45.0 |
| RIB426 | 56.9 | 41.3 | 15.4 | 43.3 | 46.7 | 33.2 |
| MDS446 | 76.5 | 59.5 | 32.2 | 29.1 | 37.3 | 31.1 |
| QAB373 | 63.3 | 41.6 | 24.4 | 33.0 | 30.6 | 27.3 |

*Example 9: Improved PD-1 binders: in vivo anti-tumor efficacy and s.c. tumor immunophenotyping*

The improved bispecific compounds of Table 25 were tested on hPD-1 transgenic mice bearing s.c. B16F10 tumors. Transgenic hPD-1 mice (C57BL/6N-Pdcd1tm1(huPDCD1-ICP11)Geno) were injected s.c. in the right flank with $1\times 10^6$ B16F10 cells. When tumors reached an average size of 70 to 100 mm$^3$ (day 0), compounds were administered i.v. at 1.25 nmoles/kg. A second administration was given on day 3. Mice were sacrificed on day 5 after study start and tumors were excised. Tumors were processed through a GentleMACS Octo Dissociator (Milteny) and cells were stained with NIR live dead stain (ThermoFisher, L10119). Cells were incubated in Fc Block (TruStain FcX™, Biolegend, 101320) 10 min before adding the surface stain (see Table 20). Cells were fixed and permeabilized with FoxP3 Staining Buffer Set (ebioscience, #00-5523-00) before intracellular staining as per Table 20. All PD-1 targeted anti-IL-2/IL-2 bispecific antibodies markedly increase the intratumoral CD8 T cell numbers, in particular PD-1+ stem-like T cells and the derivatives compared to untargeted compounds or vehicle (Table 29). CD8 T cells / Treg ratio was increased in the tumors of mice treated with bispecific compounds compared to vehicle or untargeted bispecifics.

Table 29. TILs from transgenic hPD-1 mice treated with untargeted or PD-1 targeted IL-2 antibody fusion protein as fold increase cells/gram tumor over vehicle. Last column reports CD8 to Treg cells ratios. Tumors analyzed on day 5.

| | Fold over vehicle cells/gram tumor | | | | | | | CD8/Treg ratio of cells /gram tumor |
| --- | --- | --- | --- | --- | --- | --- | --- | --- |
| Compound | Treg cells | NK cells | CD8 T cells | CD8 PD-1 + | CD8+PD-1+ stem-like | CD8+PD-1+ better effector | CD8+PD-1+TCF1- exhausted | |
| Vehicle | - | - | - | - | - | - | - | 9.3 |
| RIB426 | 8.5 | 15.8 | 25.6 | 28.1 | 22.8 | 25.5 | 40.6 | 27.9 |
| TMU471 | 20.5 | 37.0 | 107.1 | 122.7 | 121.5 | 112.7 | 156.3 | 48.4 |
| QAB373 | 13.7 | 28.0 | 79.5 | 90.2 | 72.3 | 84.0 | 122.9 | 53.6 |
| MDS446 | 10.1 | 34.6 | 105.1 | 121.2 | 75.6 | 116.0 | 167.6 | 96.2 |
| XWY176 | 6.7 | 18.1 | 41.4 | 47.7 | 38.8 | 45.7 | 59.7 | 57.2 |
| LTJ498 | 12.4 | 29.0 | 69.1 | 81.0 | 51.1 | 78.1 | 110.5 | 51.6 |
| GQM289 | 8.0 | 18.8 | 27.1 | 29.6 | 24.4 | 26.6 | 42.6 | 31.4 |
| JLI141 | 2.7 | 8.2 | 29.5 | 35.6 | 16.8 | 33.1 | 56.2 | 100.4 |
| TSQ225 | 2.2 | 12.5 | 3.3 | 1.2 | 1.4 | 1.1 | 1.3 | 14.0 |

(continued)

| | Fold over vehicle cells/gram tumor | | | | | | | CD8/Treg ratio of cells /gram tumor |
|---|---|---|---|---|---|---|---|---|
| Compound | Treg cells | NK cells | CD8 T cells | CD8 PD-1 + | CD8+PD-1+ stem-like | CD8+PD-1+ better effector | CD8+PD-1+TCF1- exhausted | |
| LNX431 | 2.8 | 18.5 | 3.6 | 1.7 | 2.0 | 1.6 | 1.7 | 12.1 |

In an efficacy study, transgenic hPD-1 mice (C57BL/6N-Pdcd1tm1(huPDCD1-ICP11)Geno) were injected s.c. in the right flank with $1 \times 10^6$ B16F10 cells. When tumors reached an average size of 70 to 100 mm$^3$ (day 0), compounds were administered i.v. at 1.25 nmoles/kg. Additional administrations were given on day 3 and 7. Tumors were measured daily and volume was calculated with the formula (length $\times$ width $\times$ width) 12. Tumor volume difference between mice treated with vehicle or the respective compound n reported as % decrease compared to vehicle. All PD-1 targeted IL-2 fusion protein compounds effectively reduced tumor volume (Table 30).

Table 30. Tumor volume difference in % to vehicle on day 7.

| Compound | % TGI vs vehicle |
|---|---|
| Vehicle | - |
| TMU471 | 59 |
| QAB373 | 58 |
| MDS446 | 80 |
| XWY176 | 69 |
| LTJ498 | 62 |
| GQM289 | 64 |
| JLI141 | 80 |
| RIB426 | 65 |

*Example 10: Stress study on anti-PD-1 , anti-IL-2/IL-2 bispecific antibodies*

Heterotetrameric bispecific antibodies XWY176, TMU471, QAB373 and MDS446 were produced with improved knob-in-hole mutations (S354C, T366W / S354C, T366S, L368A, Y407V) and Fc silencing (Table 31).

Table 31. Bispecific compounds with optimized knob-in hole mutations and Fc silencing.

| Bispecific antibody name combined to QTY065 | Sequence ID |
|---|---|
| NZA596 | 111, 095, 112, 052 |
| KTX917 | 113, 106, 114 |
| CIT348 | 115, 109, 116 |
| BGY642 | 117, 104, 112, 052 |

The four bispecific antibodies from Table 31 were exposed to thermal, pH, oxidation, freeze-thaw stress conditions. After that the compounds were tested on SEC-HPLC, iCEIF and functional ELISA Posttranslational modifications were analyzed by Mass spectrometry. The bispecific antibodies XWY176, TMU471, QAB373 and MDS446 were exposed to different stress conditions: i) incubation at 40°C for 1 or 2 weeks, ii) 3 or 4 freeze-thaw cycles, iii) in 0.1% (v/v) $H_2O_2$ for 4h or 24h, iv) low pH 3.5 for 24 or 48h, v) high pH 9.0 for 24 or 48h. To assess protein changes after the stress conditions the compounds were tested by SEC-HPLC, iCIEF, LC-MS. The functionality of the bispecific antibody was assessed with a sandwich ELISA that relies on its binding to the target antigen (hPD-1) and the integrity of the fused IL-2 via a secondary antibody (anti-IL-2 clone 5344). 60 nM of hPD-1 (ECD-His, produced in house) was coated on a Maxisorp plate (Nunc) overnight at 4°C and blocked with 5% BSA in PBS. Bispecific antibodies were added in a serial dilution in assay buffer and detected with biotinylated-5344 and Streptavidin HRP (BD Pharmingen, 554066). Absorption end signal after adding TMB was read with a plate reader (Spectramax ID3) at 450 nm. EC50 values were determined by blotting Absorbance against concentration (Graphpad Prism, sigmoidal curve fit, 4 PL, X-axis is log). Change of EC50 values for the unstressed sample (T0) was compared to the stressed samples.

All constructs showed high potential for further development based on the criteria assessed in this Example.

*Example 11: Mass spectrometry showing correct light chain pairing*

To test correct light chain pairing of the purified bispecific constructs in the IgG CrossMab or IgG kappa/lambda formats digestion by IgdE enzyme was performed and the Fab fragments were analyzed by Mass spectrometry. One single peak was visible for each of the Fabs being assayed, corresponding to the theoretical molecular weight of correctly assembled light chain and heavy chain. No incorrect light chain pairing was detected.

*Example 12: Cis-signaling in combination with PD-1 blocking antibodies*

An assay was established to assess whether an exemplary bispecific construct as disclosed herein delivers the IL-2 to CD122-CD132 on the PD-1 expressing cell bound by its anti-PD-1 arm (cis-signaling) or signals to a neighboring cell (trans). Jurkat-PD-1+ CD122+ cells were either labeled with CFSE (Invitrogen, C34557) or CTV (Invitrogen C34554). CFSE labeled cells were then exposed to 700 nm of the non-competing parent antibody to PD-1, Pembrolizumab or Nivolumab, to block PD-1 epitopes for 30 minutes at room temperature. After two washes, both CTV and CFSE labeled cells were mixed at a 1:1 ratio and activated with the bispecific immunoconjugate (1 nM) for 15 minutes at 37°C. After the stimulation period, cells were fixed immediately by adding an equal volume of Cytofix buffer (BD Biosciences 554655) for 10 minutes at 37°C. For the staining of intracellular antigens, cells were permeabilized with ice cold Perm buffer III (BD Biosciences 558050) for 15 minutes on ice. Phosphorylated STAT5 was stained using an anti-pSTAT5 pY694 antibody (clone 47/Stat5, BD Biosciences). Induction of STAT5 phosphorylation through the immunoconjugate in these cells was analyzed. The potency of the immunoconjugate is markedly reduced on cells that were pre-incubated with the parent antibody. This interference is not observed on cells were that were pre-exposed to Pembrolizumab or Nivolumab (Table 36). Furthermore, the potency of the immunoconjugate on cells that had not been exposed to any of the PD-1 binding antibodies before was the same for all samples (co-incubated with CFSE+ pre-blocked cells), indicating that the immunoconjugate signals in a cis manner, on the same cells where PD-1 binding occurs (Table 36).

Table 36. Percentage of pSTAT5 compared to non-blocked cells after stimulation with 1 nM of an exemplary immunoconjugate:

| PD1 binder | % pSTAT5 | |
|---|---|---|
| | CFSE labeled cells | CTV labeled cells |
| Non blocked | 100.00 | 100.00 |
| parent AB | 4.81 | 89.80 |
| Pembrolizumab | 82.59 | 82.56 |
| Nivolumab | 81.39 | 82.44 |

*Selected Sequences: full sequences provided in ST26 Sequence protocol:*

SEQ ID NO 006: Proleukin (artificial)

MAPTSSSTKKTQLQLEHLLLDLQMILNGINNYKNPKLTRMLTFKFYMPKKATELKHLQCLEEELKPLEEVL

NLAQSKNFHLRPRDLISNINVIVLELKGSETTFMCEYADETATIVEFLNRWITF**S**QSIISTLT

SEQ ID NO 010: Circularly permuted Proleukin variant 1 (artificial)

NFHLRPRDLISNINVIVLELKGSETTFMCEYADETATIVEFLNRWITFSQSIISTLTPTSSSTKKTQLQLE

HLLLDLQMILNGINNYKNPKLTRMLTFKFYMPKKATELKHLQCLEEELKPLEEVLNLAQSK

SEQ ID NO 011: Circularly permuted Proleukin variant 2 (artificial)

FHLRPRDLISNINVIVLELKGSETTFMCEYADETATIVEFLNRWITFSQSIISTLTPTSSSTKKTQLQLEH

LLLDLQMILNGINNYKNPKLTRMLTFKFYMPKKATELKHLQCLEEELKPLEEVLNLAQSK

SEQ ID NO 042: Antibody A VL (CDRs)

AIRLTQSPSSFSASTGDRVTITC<u>KASQSVDYQGDSYMN</u>WYQQKPGKAPKLLIY<u>AASNLESG</u>VPSRFSGSGS

GTDFTLTISSLQSEDFATYYC<u>QQSNEDPYT</u>FGGGTKVEIK

SEQ ID NO 043: Antibody A VH (CDRs)

EVQLVQSGAEVKKPGESLKISCKGS<u>GYAFTNYLIE</u>WVRQMPGKGLEWMGV<u>INPGSGGTNYNEKFKG</u>QVTIS

ADKSISTAYLQWSSLKASDTAMYYCAR<u>WRGEGYYAYFDV</u>WGQGTTVTVSS

SEQ ID NO 046: VBE401 VH (artificial): <u>CDR</u>, **IL-2CP, linkers**

EVQLVQSGAEVKKPGESLKISCKGS<u>GYAFTNYLIE</u>WVRQMPGKGLEWMGV<u>INPGSGGTNYNEKFKG</u>QVTIS

ADKSISTAYLQWSSLKASDTAMYYCAR<u>WRGE</u>**GGGFHLRPRDLISNINVIVLELKGSETTFMCEYADETATI**

**VEFLNRWITFSQSIISTLTPTSSSTKKTQLQLEHLLLDLQMILNGINNYKNPKLTRMLTFKFYMPKKATEL**

**KHLQCLEEELKPLEEVLNLAQSKGGG**<u>GYYAYFDV</u>WGQGTTVTVSS

SEQ ID NO 047: LIZ707 VH (artificial): <u>CDR</u>, **IL-2CP, linkers**

EVQLVQSGAEVKKPGESLKISCKGS<u>GYAFTNYLIE</u>WVRQMPGKGLEWMGV<u>INPG</u>**GGFYMPKKATELKHLQC**

**LEEELKPLEEVLNLAQSKNFHLRPRDLISNINVIVLELKGSETTFMCEYADETATIVEFLNRWITFCQSII**

**STLTPTSSSTKKTQLQLEHLLLDLQMILNGINNYKNPKLTRMLTFKGGG**<u>SGGTNYNEKFKG</u>QVTISADKSI

STAYLQWSSLKASDTAMYYCAR<u>WRGEGYYAYFDV</u>WGQGTTVTVSS

SEQ ID NO 052: **QTY065** LC <u>CDR</u>, **IL-2CP, linkers,** *constant*

AIRLTQSPSSFSASTGDRVTITCKASQSVDY**GGGNFHLRPRDLISNINVIVLELKGSETTFMCEYADETAT**

**IVEFLNRWITFSQSIISTLTPTSSSTKKTQLQLEHLLLDLQMILNGINNYKNPKLTRMLTFKFYMPKKATE**

**LKHLQCLEEELKPLEEVLNLAQSKGGGG**DSYMNWYQQKPGKAPKLLIYAASNLESGVPSRFSGSGSGTDFT

LTISSLQSEDFATYYCQQSNEDPYTFGGGTKVEIK*RTVAAPSVFIFPPSDEQLKSGTASVVCLLNNFYPRE*

*AKVQWKVDNALQSGNSQESVTEQDSKDSTYSLSSTLTLSKADYEKHKVYACEVTHQGLSSPVTKSFNRGEC*
SEQ ID NO 085: 21A08Ap1, 21A08Ap2 VH (CDRs)

EVQLLESGGGLVQPGGSLRLSCAASGFTFSINAMTWVRQAPGKGLEWVSTISGSGFSTYYADSLKGRFTIS

RDNSKNTLYLQMNSLRAEDTAVYYCAKEVYGDYWGQGTLVTVSS
SEQ ID NO 086: 21A08Ap1 VL (CDRs)

RSVLTQPPSASGTPGQRVSISCSGASSNIGSQSVFWYQQLPGTAPKLLIYSNNQRPSGVPDRFSGSKSGTS

ASLAISGLRSEDEADYYCAAWDDSLSIWVFGGGTKLTVL
SEQ ID NO 087: 21A08Ap2 VL (CDRs)

RSVLTQPPSASGTPGQRVSISCSGASSNIGSSSVFWYQQLPGTAPKLLIYSNNQRPSGVPDRFSGSKSGTS

ASLAISGLRSEDEADYYCAAWDDSLSIWVFGGGTKLTVL
SEQ ID NO 091: 21A08Ap3 VL (CDRs)

RSVLTQPPSASGTPGQRVSISCSGASSNIGSNAVFWYQQLPGTAPKLLIYSNNQRPSGVPDRFSGSKSGTS

ASLAISGLRSEDEADYYCAAWDDSLSIWVFGGGTKLTVL
SEQ ID NO 094 IgG scFv N-terminal LTJ498 HC: *VH (CDR)* - constant

*EVQLLESGGGLVQPGGSLRLSCAASGFTFSINAMTWVRQAPGKGLEWVSTISGSGFSTYYADSLKGRFTIS*

*RDNSKNTLYLQMNSLRAEDTAVYYCAKEVYGDYWGQGTLVTVSS*ASTKGPSVFPLAPSSKSTSGGTAALGC

LVKDYFPEPVTVSWNSGALTSGVHTFPAVLQSSGLYSLSSVVTVPSSSLGTQTYICNVNHKPSNTKVDKRV

EPKSCDKTHTCPPCPAPEAAGGPSVFLFPPKPKDTLMISRTPEVTCVVVDVSHEDPEVKFNWYVDGVEVHN

AKTKPREEQYNSTYRVVSVLTVLHQDWLNGKEYKCKVSNKALAAPIEKTISKAKGQPREPQVYTLPPSREE

MTKNQVSLTCLVKGFYPSDIAVEWESNGQPENNYKTTPPVLDSDGSFFLTSKLTVDKSRWQQGNVFSCSVM

HEALHNHYTQKSLSLSPGK
SEQ ID NO 095 IgG scFv N-terminal LTJ498 LC: *VL (CDR)* , constant

EP 4 431 525 A1

36

*RSVLTQPPSASGTPGQRVSISCSGASSNIGSQSVFWYQQLPGTAPKLLIYSNNQRPSGVPDRFSGSKSGTS*

*ASLAISGLRSEDEADYYCAAWDDSLSIWVFGGGTKLTVLG*QPKAAPSVTLFPPSSEELQANKATLVCLISD

FYPGAVTVAWKADSSPVKAGVETTTPSKQSNNKYAASSYLSLTPEQWKSHRSYSCQVTHEGSTVEKTVAPT

ECS

SEQ ID NO 097 IgG scFv N-terminal JLI141 HC: *VH (CDR)*, constant

*EVQLLESGGGLVQPGGSLRLSCAASGFTFSINAMTWVRQAPGKGLEWVSTISGSGFSTYYADSLKGRFTIS*

*RDNSKNTLYLQMNSLRAEDTAVYYCAKEVYGDYWGQGTLVTVSS*ASTKGPSVFPLAPSSKSTSGGTAALGC

LVKDYFPEPVTVSWNSGALTSGVHTFPAVLQSSGLYSLSSVVTVPSSSLGTQTYICNVNHKPSNTKVDKRV

EPKSCDKTHTCPPCPAPEAAGGPSVFLFPPKPKDTLMISRTPEVTCVVVDVSHEDPEVKFNWYVDGVEVHN

AKTKPREEQYNSTYRVVSVLTVLHQDWLNGKEYKCKVSNKALAAPIEKTISKAKGQPREPQVYTLPPSREE

MTKNQVSLTCLVKGFYPSDIAVEWESNGQPENNYKTTPPVLDSDGSFFLTSKLTVDKSRWQQGNVFSCSVM

HEALHNHYTQKSLSLSPGK

SEQ ID NO 098 IgG scFv N-terminal JLI141 LC: *VL (CDR)*, constant

*RSVLTQPPSASGTPGQRVSISCSGASSNIGSSSVFWYQQLPGTAPKLLIYSNNQRPSGVPDRFSGSKSGTS*

*ASLAISGLRSEDEADYYCAAWDDSLSIWVFGGGTKLTVLG*QPKAAPSVTLFPPSSEELQANKATLVCLISD

FYPGAVTVAWKADSSPVKAGVETTTPSKQSNNKYAASSYLSLTPEQWKSHRSYSCQVTHEGSTVEKTVAPT

ECS

SEQ ID NO 100 IgG k/l XWY176 QTY065 HC: *VH(CDR)* constant

*EVQLVQSGAEVKKPGESLKISCKGSGYAFTNYLIEWVRQMPGKGLEWMGVINPGSGGTNYNEKFKGQVTIS*

*ADKSISTAYLQWSSLKASDTAMYYCARWRGEGYYAYFDVWGQGTTVTVSS*ASTKGPSVFPLAPSSKSTSGG

TAALGCLVKDYFPEPVTVSWNSGALTSGVHTFPAVLQSSGLYSLSSVVTVPSSSLGTQTYICNVNHKPSNT

KVDKRVEPKSCDKTHTCPPCPAPEAAGGPSVFLFPPKPKDTLMISRTPEVTCVVVDVSHEDPEVKFNWYVD

GVEVHNAKTKPREEQYNSTYRVVSVLTVLHQDWLNGKEYKCKVSNKALAAPIEKTISKAKGQPREPQVYTL

PPSREEMTKNQVSLYCLVKGFYPSDIAVEWESNGQPENNYKTTPPVLDSDGSFFLYSKLTVDKSRWQQGNV

FSCSVMHEALHNHYTQKSLSLSPGK

SEQ ID NO 104: Crossmab QAB373 XVT458-z2-m6 LC with crossmab switch: *VL(CDR)* - CH1

(continued)

*DIQMTQSPSSVSASVGDRVTITCSASQGISGDLNWYQQKPGKAPKLLIYHTSQLHSDVPSRFSGSGSGTDF*

*TLTISSLQPEDFATYYCQGYSKDLLTFGGGTKLEIK*SSASTKGPSVFPLAPSSKSTSGGTAALGCLVKDYF

PEPVTVSWNSGALTSGVHTFPAVLQSSGLYSLSSVVTVPSSSLGTQTYICNVNHKPSNTKVDKRVEPKSC

SEQ ID NO 106 IgG scFv N-terminal TMU471 LC: *VL(CDR)* - CL(kappa)

*DIQMTQSPSSVSASVGDRVTITCSASQGISGDLNWYQQKPGKAPKLLIYHTSQRHSDVPSRFSGSGSGTDF*

*TLTISSLQPEDFATYYCQGYSKDLLTFGGGTKLEIK*RTVAAPSVFIFPPSDEQLKSGTASVVCLLNNFYPR

EAKVQWKVDNALQSGNSQESVTEQDSKDSTYSLSSTLTLSKADYEKHKVYACEVTHQGLSSPVTKSFNRGE

C

SEQ ID NO 109 IgG scFv N-terminal MDS446LC: *VL(CDR)* - CL(kappa)

*DIQMTQSPSSVSASVGDRVTITCSASQGISGDLNWYQQKPGKAPKLLIYHTSQLHSDVPSRFSGSGSGTDF*

*TLTISSLQPEDFATYYCQGYSKDLLTFGGGTKLEIK*RTVAAPSVFIFPPSDEQLKSGTASVVCLLNNFYPR

EAKVQWKVDNALQSGNSQESVTEQDSKDSTYSLSSTLTLSKADYEKHKVYACEVTHQGLSSPVTKSFNRGE

C

SEQ ID NO 111 21A08Ap1 HC (NZA596): *VH(CDR)*, constant

*EVQLLESGGGLVQPGGSLRLSCAASGFTFSINAMTWVRQAPGKGLEWVSTISGSGFSTYYADSLKGRFTIS*

*RDNSKNTLYLQMNSLRAEDTAVYYCAKEVYGDYWGQGTLVTVSS*ASTKGPSVFPLAPSSKSTSGGTAALGC

LVKDYFPEPVTVSWNSGALTSGVHTFPAVLQSSGLYSLSSVVTVPSSSLGTQTYICNVNHKPSNTKVDKRV

EPKSCDKTHTCPPCPAPE**AA**GGPSVFLFPPKPKDTLMISRTPEVTCVVVDVSHEDPEVKFNWYVDGVEVHN

AKTKPREEQYNSTYRVVSVLTVLHQDWLNGKEYKCKVSNKAL**A**APIEKTISKAKGQPREPQVCTLPPSREE

MTKNQVSLSCAVKGFYPSDIAVEWESNGQPENNYKTTPPVLDSDGSFFLVSKLTVDKSRWQQGNVFSCSVM

HEALHNHYTQKSLSLSPGK

SEQ ID NO 112 QTY065 HC (NZA596): *VH(CDR)*, constant

*EVQLVQSGAEVKKPGESLKISCKGSGYAFTNYLIEWVRQMPGKGLEWMGVINPGSGGTNYNEKFKGQVTIS*

*ADKSISTAYLQWSSLKASDTAMYYCARWRGEGYYAYFDVWGQGTTVTVSS*ASTKGPSVFPLAPSSKSTSGG

TAALGCLVKDYFPEPVTVSWNSGALTSGVHTFPAVLQSSGLYSLSSVVTVPSSSLGTQTYICNVNHKPSNT

KVDKRVEPKS**C**DKTHTCPPCPAPE**AA**GGPSVFLFPPKPKDTLMISRTPEVTCVVVDVSHEDPEVKFNWYVD

GVEVHNAKTKPREEQYNSTYRVVSVLTVLHQDWLNGKEYKCKVSNKAL**A**APIEKTISKAKGQPREPQVYTL

PPCREEMTKNQVSLWCLVKGFYPSDIAVEWESNGQPENNYKTTPPVLDSDGSFFLYSKLTVDKSRWQQGNV

FSCSVMHEALHNHYTQKSLSLSPGK

SEQ ID NO 113 XVT-z2-m3 HC (KTX917): *VH(CDR)* - constant

*QVQLVQSGAEVKKPGASVKVSCKASGYTFTNFYIHWVRQAPGQGLEWMGRIYPNYGITAYNQKFKDRVTMT*

*VDTSTSTAYMELRSLRSDDTAVYYCARGYSYAMDYWGQGTTVTVSS*ASTKGPSVFPLAPSSKSTSGGTAAL

GCLVKDYFPEPVTVSWNSGALTSGVHTFPAVLQSSGLYSLSSVVTVPSSSLGTQTYICNVNHKPSNTKVDK

RVEPKSCDKTHTCPPCPAPE**AA**GGPSVFLFPPKPKDTLMISRTPEVTCVVVDVSHEDPEVKFNWYVDGVEV

HNAKTKPREEQYNSTYRVVSVLTVLHQDWLNGKEYKCKVSNKAL**A**APIEKTISKAKGQPREPQVCTLPPSR

EEMTKNQVSLSCAVKGFYPSDIAVEWESNGQPENNYKTTPPVLDSDGSFFLVSKLTVDKSRWQQGNVFSCS

VMHEALHNHYTQKSLSLSPGK

SEQ ID NO 114 XVT-z2-m3 HC -IL-2 scFv (KTX917): *AbAVL*(**IL-2**), *AbAVH*, z2-XVT458-m3 VH,

(continued)

*AIRLTQSPSSFSASTGDRVTITCKASQSVDY***GGG**N**FHLRPRDLISNINVIVLELKGSETTFMCEYADETAT**

**IVEFLNRWITFSQSIISTL**TPT**SSSTKKTQLQLEHLLLDLQMILNGINNYKNPKLTRMLTFKFYMPKKATE**

**LKHLQCLEEELKPLEEVLNLAQSK**GGGG*DSYMNWYQQKPGKAPKLLIYAASNLESGVPSRFSGSGSGTDFT*

*LTISSLQSEDFATYYCQQSNEDPYTFGGGTKVEIK***GGGGSGGGGSGGGGS**EVQLVQSGAEVKKPGESLKIS

CKGSGYAFTNYLIEWVRQMPGKGLEWMGVINPGSGGTNYNEKFKGQVTISADKSISTAYLQWSSLKASDTA

MYYCARWRGEGYYAYFDVWGQGTTVTVSS**GGGGSGGGGS**QVQLVQSGAEVKKPGASVKVSCKASGYTFTNF

YIHWVRQAPGQGLEWMGRIYPNYGITAYNQKFKDRVTMTVDTSTSTAYMELRSLRSDDTAVYYCARGYSYA

MDYWGQGTTVTVSSASTKGPSVFPLAPSSKSTSGGTAALGCLVKDYFPEPVTVSWNSGALTSGVHTFPAVL

QSSGLYSLSSVVTVPSSSLGTQTYICNVNHKPSNTKVDKRVEPKS**C**DKTHTCPPCPAPE**AA**GGPSVFLFPP

KPKDTLMISRTPEVTCVVVDVSHEDPEVKFNWYVDGVEVHNAKTKPREEQYNSTYRVVSVLTVLHQDWLNG

KEYKCKVSNKAL**A**APIEKTISKAKGQPREPQVYTLPPCREEMTKNQVSLWCLVKGFYPSDIAVEWESNGQP

ENNYKTTPPVLDSDGSFFLYSKLTVDKSRWQQGNVFSCSVMHEALHNHYTQKSLSLSPGK

SEQ ID NO 115: XVT-z2-m6 HC (CIT348)

*QVQLVQSGAEVKKPGASVKVSCKASGYTF*TNFYIH*WVRQAPGQGLEWMGS*IYPNYGITAYNQKFKDRVTMT

*VDTSTSTAYMELRSLRSDDTAVYYCAR*GYSYAMD*YWGQGTTVTVSS*ASTKGPSVFPLAPSSKSTSGGTAAL

GCLVKDYFPEPVTVSWNSGALTSGVHTFPAVLQSSGLYSLSSVVTVPSSSLGTQTYICNVNHKPSNTKVDK

RVEPKSCDKTHTCPPCPAPEAAGGPSVFLFPPKPKDTLMISRTPEVTCVVVDVSHEDPEVKFNWYVDGVEV

HNAKTKPREEQYNSTYRVVSVLTVLHQDWLNGKEYKCKVSNKALAAPIEKTISKAKGQPREPQVCTLPPSR

EEMTKNQVSLSCAVKGFYPSDIAVEWESNGQPENNYKTTPPVLDSDGSFFLVSKLTVDKSRWQQGNVFSCS

VMHEALHNHYTQKSLSLSPGK

SEQ ID NO 116: XVT-z2-m3 HC -IL-2 scFv (CIT348): *AbAVL*(**IL-2**), *AbAVH*, z2-XVT458-m6 VH,

(continued)

*AIRLTQSPSSFSASTGDRVTITCKASQSVDY***GGG**NFHLRPRDLISNINVIVLELKGSETTFMCEYADETAT

IVEFLNRWITFSQSIISTLTPTSSSTKKTQLQLEHLLLDLQMILNGINNYKNPKLTRMLTFKFYMPKKATE

LKHLQCLEEELKPLEEVLNLAQSKGGGG*DSYMNWYQQKPGKAPKLLIYAASNLESGVPSRFSGSGSGTDFT*

*LTISSLQSEDFATYYCQQSNEDPYTFGGGTKVEIK***GGGGSGGGGSGGGGS***EVQLVQSGAEVKKPGESLKIS*

*CKGSGYAFTNYLIEWVRQMPGKGLEWMGVINPGSGGTNYNEKFKGQVTISADKSISTAYLQWSSLKASDTA*

*MYYCARWRGEGYYAYFDVWGQGTTVTVSS***GGGGSGGGGS***QVQLVQSGAEVKKPGASVKVSCKASGYTFTNF*

*YIHWVRQAPGQGLEWMGSIYPNYGITAYNQKFKDRVTMTVDTSTSTAYMELRSLRSDDTAVYYCARGYSYA*

*MDYWGQGTTVTVSS*ASTKGPSVFPLAPSSKSTSGGTAALGCLVKDYFPEPVTVSWNSGALTSGVHTFPAVL

QSSGLYSLSSVVTVPSSSLGTQTYICNVNHKPSNTKVDKRVEPKS**C**DKTHTCPPCPAPE**AA**GGPSVFLFPP

KPKDTLMISRTPEVTCVVVDVSHEDPEVKFNWYVDGVEVHNAKTKPREEQYNSTYRVVSVLTVLHQDWLNG

KEYKCKVSNKAL**AA**PIEKTISKAKGQPREPQVYTLPPCREEMTKNQVSLWCLVKGFYPSDIAVEWESNGQP

ENNYKTTPPVLDSDGSFFLYSKLTVDKSRWQQGNVFSCSVMHEALHNHYTQKSLSLSPGK

SEQ ID NO 117 XVT-z2-m6 HC Crossmab (BGY642); *VH* (CDR)-CLk-Hinge-CH2-CH3

*QVQLVQSGAEVKKPGASVKVSCKASGYTFTNFYIHWVRQAPGQGLEWMGSIYPNYGITAYNQKFKDRVTMT*

*VDTSTSTAYMELRSLRSDDTAVYYCARGYSYAMDYWGQGTTVTVSS*ASVAAPSVFIFPPSDEQLKSGTASV

VCLLNNFYPREAKVQWKVDNALQSGNSQESVTEQDSKDSTYSLSSTLTLSKADYEKHKVYACEVTHQGLSS

PVTKSFNRGECDKTHTCPPCPAPE**AA**GGPSVFLFPPKPKDTLMISRTPEVTCVVVDVSHEDPEVKFNWYVD

GVEVHNAKTKPREEQYNSTYRVVSVLTVLHQDWLNGKEYKCKVSNKAL**A**APIEKTISKAKGQPREPQV**C**TL

PPSREEMTKNQVSL**SCA**VKGFYPSDIAVEWESNGQPENNYKTTPPVLDSDGSFFL**V**SKLTVDKSRWQQGNV

FSCSVMHEALHNHYTQKSLSLSPGK

| | |
|---|---|
| SEQ ID NO 118: | HCDR1 GFTFSINAMT |
| SEQ ID NO 119: | HCDR2 *TISGSGFSTYYADSLKGR* |
| SEQ ID NO 120: | HCDR3 EVYGDY |
| SEQ ID NO 121: | LCDR1 SGX$_1$SSNIGS(XX)$_2$VF wherein X$_1$ is N, S, Q, or A, and where XX$_2$ is NS, QS, SS, or NA |
| SEQ ID NO 122: | LCDR2 SNNQRPS |
| SEQ ID NO 123: | LCDR3 AAWDDSLSIWVF |
| SEQ ID NO 124: | LCDR1 SGASSNIGS**QS**VF |

(continued)

| | | |
|---|---|---|
| SEQ ID NO 125: | LCDR1 | SGASSNIGSS**SS**VF |
| SEQ ID NO 126: | LCDR1 | SGASSNIGS**NA**VF |
| SEQ ID NO 127: | HCDR1 | NFYIH |
| SEQ ID NO 128: | HCDR2 | **R**IYPNYGITAYNQKFKD |
| SEQ ID NO 129: | HCDR3 | GYSYAMDY |
| SEQ ID NO 130: | LCDR1 | SASQGISGDLN |
| SEQ ID NO 131: | LCDR2 | HTS**QR**HS |
| SEQ ID NO 132: | LCDR3 | Q**G**YSKDLLT |
| SEQ ID NO 133: | HCDR2 | SIYPNYGITAYNQKFKD |

**Claims**

1. An immunoconjugate comprising

   i. an anti-PD1 antigen binding domain, comprising

   - an antibody heavy chain variable domain polypeptide (PD1-VH), and
   - an antibody light chain variable domain polypeptide (PD1-VL);

   ii. an anti-IL-2 antigen binding domain, comprising

   - an antibody heavy chain variable domain polypeptide (IL2-VH) having from N' to C' terminus the following domains: a first framework region (IL2VH$_1$), a first complementarity determining region (IL2HCDR1), a second framework region (IL2VH$_2$), a second complementarity determining region (IL2HCDR2), a third framework region (IL2VH$_3$), a third complementarity determining region (IL2HCDR3), and a fourth framework region (IL2VH$_4$); and
   - an antibody light chain variable domain polypeptide (IL2-VL) having from N' to C' terminus the following domains: a first framework region (IL2VL$_1$), a first complementarity determining region (IL2LCDR1), a second framework region (IL2VL$_2$), a second complementarity determining region (IL2LCDR2), a third framework region (IL2VL$_3$), a third complementarity determining region (an IL2LCDR3), and a fourth framework region (IL2VL$_4$); and

   iii. an IL-2 polypeptide

   wherein the IL-2 polypeptide is covalently linked to the IL2-VL, or the IL2-VH, to provide a single contiguous recombinant polypeptide;
   **characterized in that**
   binding of pembrolizumab, or nivolumab to a PD-1+ cell does not significantly inhibit binding of the immunoconjugate to said cell.

2. The immunoconjugate according to claim 1, wherein

   - the PD1-VH comprises an HCDR1 having the sequence GFTFSINAMT (SEQ ID NO 118), an HCDR2 having the sequence TISGSGFSTYYADSLKGR (SEQ ID NO 119), and an HCDR3 having the sequence EVYGDY (SEQ ID NO 120); and
   - the PD1-VL comprises an LCDR1 having the sequence SGX$_1$SSNIGS(XX)$_2$VF (SEQ ID NO 121), an LCDR2 having the sequence SNNQRPS (SEQ ID NO 122), and an LCDR3 having the sequence AAWDDSLSIWVF (SEQ ID NO 123);
   wherein X$_1$ is N, S, Q, or A;
   and wherein (XX)$_2$ is NS, QS, SS, or NA.

3. The immunoconjugate according to claim 2, wherein the PD1-VL comprises an **LCDR1** having a sequence selected from the group of:

   a. SGASSNIGS**QS**VF (SEQ ID NO 124);
   b. SGASSNIGS**SS**VF (SEQ ID NO 125); or
   c. SGASSNIGS**NA**VF (SEQ ID NO 126).

4. The immunoconjugate according to claim 2 or 3, wherein:

   a. the PD1-VH comprises, or consists of a polypeptide having the sequence SEQ ID NO 085 and the PD1-VL comprises, or consists of a polypeptide having the sequence SEQ ID NO 086; or
   b. the PD1-VH comprises, or consists of a polypeptide having the sequence SEQ ID NO 085 and the PD1-VL comprises, or consists of a polypeptide having the sequence SEQ ID NO 087; or
   c. the PD1-VH comprises, or consists of a polypeptide having the sequence SEQ ID NO 085 VH 21A08 and the PD1-VL comprises, or consists of a polypeptide having the sequence SEQ ID NO 91.

5. The immunoconjugate according to any one of the claims 1 to 4, wherein

- the PD1-VH comprises, or consists of a polypeptide at least ($\geq$) 95%, 98%, 99% similar to SEQ ID NO 085, and the PD1-VL comprises, or consists of a polypeptide $\geq$ 95%, 98%, 99% similar to SEQ ID NO 086;
- the PD1-VH comprises, or consists of a polypeptide a polypeptide $\geq$ 95%, 98%, 99% similar to SEQ ID NO 085 and the PD1-VL comprises, or consists of a polypeptide $\geq$ 95%, 98%, 99% similar to SEQ ID NO 087;
- the PD1-VH comprises, or consists of a polypeptide a polypeptide $\geq$ 95%, 98%, 99% similar to SEQ ID NO 085; and the PD1-VL comprises, or consists of a polypeptide $\geq$ 95%, 98%, 99% similar to SEQ ID NO 091;

and wherein the affinity constant ($K_D$) of the immunoconjugate for PD-1 is in the range of $1.0 \times 10^{-9}$ to $1.5 \times 10^{-11}$ mol/L as measured according to the protocol provided in Example 2.

6. The immunoconjugate according to claim 1, wherein

a. the PD1-VH comprises an HCDR1 having the sequence NFYIH (SEQ ID NO 127), an HCDR2 having the sequence **R**IYPNYGITAYNQKFKD (SEQ ID NO 128), and an HCDR3 having the sequence GYSYAMDY (SEQ ID NO 129); and the PD1-VL comprises an LCDR1 having the sequence SASQGISGDLN (SEQ ID NO 130), an LCDR2 having the sequence HTS**QR**HS (SEQ ID NO 131), and an LCDR3 having the sequence Q**G**YSKDLLT (SEQ ID NO 132); or
b. the PD1-VH comprises an HCDR1 having the sequence NFYIH (SEQ ID NO 127), an HCDR2 having the sequence SIYPNYG**I**TAYNQKFKD (SEQ ID NO 133), and an HCDR3 having the sequence GYSYAMDY (SEQ ID NO 129); and the PD1-VL comprises an LCDR1 having the sequence SASQGISGDLN (SEQ ID NO 130), an LCDR2 having the sequence HTS**Q**LHS (SEQ ID NO 134), and an LCDR3 having the sequence Q**G**YSKDLLT (SEQ ID NO 132).

7. The immunoconjugate according to any one of the claims 1 to 8, wherein the IL-2 polypeptide is a circularly permuted IL-2 (IL2CP) polypeptide;

wherein the IL2CP is >98% similar to SEQ ID NO 010 or SEQ ID NO 011; and
the IL2CP N'-terminus and IL2CP C'-terminus are covalently linked, optionally by means of one, or two peptide linkers, to amino acid residues within an IL2LCDR1 domain.

8. The immunoconjugate according to any one of the claims 1 to 16, wherein the IL2-VH is more than >98%, or 99% identical to SEQ ID NO 043, and the IL2-VL is more than >98%, or 99% identical to SEQ ID NO 042; and wherein the $K_D$ of an antibody **characterized by** the IL-2VH and the IL2-VL for an IL-2 polypeptide having SEQ ID NO 006 is in the range of 270 nM to 2 nM.

9. The immunoconjugate according to any one of the claims 1 to 8, wherein the anti-IL-2 antigen binding domain comprises, or consists of, **an IL2-VL having sequence SEQ ID NO 042,** associated with an IL2-VH selected from SEQ ID NO 046, or 047.

10. The immunoconjugate according to any one of claims 1 to 9, wherein the immunoconjugate comprises an **IgG Fc portion.**

11. The immunoconjugate according to claim 10, wherein the IgG Fc portion is **characterized by** the presence of one or more modifications to constant regions of the heavy chains to enhance correct heavy chain pairing, particularly a set of know and hole modifications selected from:

- knob: S354C, T366W and hole: Y349C, T366S, L368A, Y407V;
- knob: T366Y, and hole Y407T;
- knob: Y349C, T366W, and hole: S354C, T366S, L368A, Y407V; or
- knob: T366W, and Hole: Y407A, T366S, L368A.

12. The immunoconjugate according to any one of the claims 1 to 11, wherein the immunoconjugate is a **heterotetrameric** IgG consisting of

- a first heterodimer comprising the anti-PD-1 binding domain as specified in any in of the claims 1 to 8; and
- a second heterodimer comprising the anti-IL2 binding domain as specified in any in of the claims 1, or 9 to 21.

13. The immunoconjugate according to any one of the claims 1 to 12, wherein the immunoconjugate comprises, or consists of the polypeptides having the sequences:

    a. SEQ ID NO 111, SEQ ID NO 095, SEQ ID NO 112, and SEQ ID NO 052 (NZA596);
    b. SEQ ID NO 117, SEQ ID NO 104, SEQ ID NO 112, and SEQ ID NO 091 (BGY642);
    c. SEQ ID NO 113, 106, and 114 (KTX917);
    d. SEQ ID NO 115, 109, and 116 (CIT348);
    e. SEQ ID NO 094, SEQ ID NO 095, SEQ ID NO 100, and SEQ ID NO 052 (XWY176);
    f. SEQ ID NO 097, SEQ ID NO 098, SEQ ID NO 100, and SEQ ID NO 052 (GQM289).

14. An isolated nucleic acid encoding the immunoconjugate according to any one of claims 1 to 13;
particularly wherein the isolated nucleic acid is comprised in a mammalian expression vector under control of a promoter operable in a mammalian cell.

15. A combination medicament comprising

    - the immunoconjugate as specified in any one of the claims 1 to 13, and
    - an anti-PD-1 antagonist antibody selected from the list consisting of nivolumab, pembrolizumab, dostarlimab, sintilimab, tislelizumab, cemiplimab, cetrelimab, sasanlimab, particularly nivolumab or pembrolizumab.

Fig. 1

**VL1 / CDR-L1**

| Region | Structure numbering | Kabat numbering |
|---|---|---|
| VL1 | 1 | 1 |
| VL1 | 2 | 2 |
| VL1 | 3 | 3 |
| VL1 | 4 | 4 |
| VL1 | 5 | 5 |
| VL1 | 6 | 6 |
| VL1 | 7 | 7 |
| VL1 | 8 | 8 |
| VL1 | 9 | 9 |
| VL1 | 10 | 10 |
| VL1 | 11 | 11 |
| VL1 | 12 | 12 |
| VL1 | 13 | 13 |
| VL1 | 14 | 14 |
| VL1 | 15 | 15 |
| VL1 | 16 | 16 |
| VL1 | 17 | 17 |
| VL1 | 18 | 18 |
| VL1 | 19 | 19 |
| VL1 | 20 | 20 |
| VL1 | 21 | 21 |
| VL1 | 22 | 22 |
| VL1 | 23 | 23 |
| CDR-L1 | 24 | 25 |
| CDR-L1 | 25 | 26 |
| CDR-L1 | 26 | 27 |
| CDR-L1 | 27 | 27 |
| CDR-L1 | 28 | 27a |
| CDR-L1 | 29 | 27b |
| CDR-L1 | 30 | 27c |
| CDR-L1 | 31 | 27d |
| CDR-L1 | 32 | 27e |
| CDR-L1 | 33 | 27f |

**VL3**

| Region | Structure numbering | Kabat numbering |
|---|---|---|
| VL3 | 63 | 57 |
| VL3 | 64 | 58 |
| VL3 | 65 | 59 |
| VL3 | 66 | 60 |
| VL3 | 67 | 61 |
| VL3 | 68 | 62 |
| VL3 | 69 | 63 |
| VL3 | 70 | 64 |
| VL3 | 71 | 65 |
| VL3 | 72 | 66 |
| VL3 | 73 | 67 |
| VL3 | 74 | 68 |
| VL3 | 75 | 69 |
| VL3 | 76 | 70 |
| VL3 | 77 | 71 |
| VL3 | 78 | 72 |
| VL3 | 79 | 73 |
| VL3 | 80 | 74 |
| VL3 | 81 | 75 |
| VL3 | 82 | 76 |
| VL3 | 83 | 77 |
| VL3 | 84 | 78 |
| VL3 | 85 | 79 |
| VL3 | 86 | 80 |
| VL3 | 87 | 81 |
| VL3 | 88 | 82 |
| VL3 | 89 | 83 |
| VL3 | 90 | 84 |
| VL3 | 91 | 85 |
| VL3 | 92 | 86 |
| VL3 | 93 | 87 |
| VL3 | 94 | 88 |
| VL3 | 95 | 89 |

**VL2 / CDR-L2**

| Region | Structure numbering | Kabat numbering |
|---|---|---|
| VL2 | 34 | 28 |
| VL2 | 35 | 29 |
| VL2 | 36 | 30 |
| VL2 | 37 | 31 |
| VL2 | 38 | 32 |
| VL2 | 39 | 33 |
| VL2 | 40 | 34 |
| VL2 | 41 | 35 |
| VL2 | 42 | 36 |
| VL2 | 43 | 37 |
| VL2 | 44 | 38 |
| VL2 | 45 | 39 |
| VL2 | 46 | 40 |
| VL2 | 47 | 41 |
| VL2 | 48 | 42 |
| VL2 | 49 | 43 |
| VL2 | 50 | 44 |
| VL2 | 51 | 45 |
| VL2 | 52 | 46 |
| VL2 | 53 | 47 |
| VL2 | 54 | 48 |
| VL2 | 55 | 49 |
| CDR-L2 | 56 | 50 |
| CDR-L2 | 57 | 51 |
| CDR-L2 | 58 | 52 |
| CDR-L2 | 59 | 53 |
| CDR-L2 | 60 | 54 |
| CDR-L2 | 61 | 55 |
| CDR-L2 | 62 | 56 |

**CDR-L3 / VL4**

| Region | Structure numbering | Kabat numbering |
|---|---|---|
| CDR-L3 | 96 | 90 |
| CDR-L3 | 97 | 91 |
| CDR-L3 | 98 | 92 |
| CDR-L3 | 99 | 93 |
| CDR-L3 | 100 | 94 |
| CDR-L3 | 101 | 95 |
| CDR-L3 | 102 | 95a |
| CDR-L3 | 103 | 95b |
| CDR-L3 | 104 | 95c |
| CDR-L3 | 105 | 95d |
| CDR-L3 | 106 | 95e |
| CDR-L3 | 107 | 95f |
| CDR-L3 | 108 | 96 |
| CDR-L3 | 109 | 97 |
| VL4 | 120 | 98 |
| VL4 | 121 | 99 |
| VL4 | 122 | 100 |
| VL4 | 123 | 101 |
| VL4 | 124 | 102 |
| VL4 | 125 | 103 |
| VL4 | 126 | 104 |
| VL4 | 127 | 105 |
| VL4 | 128 | 106 |
| VL4 | 129 | 107 |

Fig. 1 (continued)

| Region | VH1 | | | | | | | | | | | | | | | | | | | | | | | |
|---|---|---|---|---|---|---|---|---|---|---|---|---|---|---|---|---|---|---|---|---|---|---|---|---|
| Structure numbering | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
| Kabat numbering | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |

| Region | VH3 | | | | | | | | | | | | | | | | | | | | | | | |
|---|---|---|---|---|---|---|---|---|---|---|---|---|---|---|---|---|---|---|---|---|---|---|---|---|
| Structure numbering | 72 | 73 | 74 | 75 | 76 | 77 | 78 | 79 | 80 | 81 | 82 | 83 | 84 | 85 | 86 | 87 | 88 | 89 | 90 | 91 | 92 | 93 | 94 | 95 |
| Kabat numbering | 67 | 68 | 69 | 70 | 71 | 72 | 73 | 74 | 75 | 76 | 77 | 78 | 79 | 80 | 81 | 82 | 83 | 84 | 85 | 86 | 87 | 88 | 89 | 90 |

| CDR-H1 | | | | | | | | | | | | | | | | | VH2 | | | | | | | | | | CDR-H2 | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| 25 | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 48 | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 |
| 25 | 26 | 27 | 28 | 29 | 30 | 31 | 32 | 33 | 34 | 35 | 35a | 35b | 36 | 37 | 38 | 39 | 40 | 41 | 42 | 43 | 44 | 45 | 46 | 47 | 48 | 49 | 50 | 51 | 52 | 52a | 52b | 52c | 53 | 54 | 55 | 56 | 57 | 58 | 59 | 60 | 61 | 62 | 63 | 64 | 65 | 66 |

| | | | | | | | | CDR-H3 | | | | | | | | | | | | | | VH4 | | | | | | | | | |
|---|---|---|---|---|---|---|---|---|---|---|---|---|---|---|---|---|---|---|---|---|---|---|---|---|---|---|---|---|---|---|---|
| 96 | 97 | 98 | 99 | 100 | 101 | 102 | 103 | 104 | 105 | 106 | 107 | 108 | 109 | 110 | 111 | 112 | 113 | 114 | 115 | 116 | 117 | 118 | 119 | 120 | 121 | 122 | 123 | 124 | 125 | 126 | 127 |
| 91 | 92 | 93 | 94 | 95 | 96 | 97 | 98 | 99 | 100 | 100a | 100b | 100c | 100d | 100e | 100f | 100g | 100h | 100i | 101 | 102 | 103 | 104 | 105 | 106 | 107 | 108 | 109 | 110 | 111 | 112 | 113 |

Fig. 2

Antibody / IL-2 complex

Antibody fusion
protein on light chain

Antibody fusion
protein on heavy chain

Fig. 3.

Fig. 4

**A.** Double scFv-fusion

**B.** Fab-double scFv

**C.** IgG format with CrossMab$^{CH1-CL}$

**D.** IgG with C-terminal scFv

**E.** IgG with N-terminal scFv

**F.** IgG format kappa/lambda

Fig. 5

# EP 4 431 525 A1

Europäisches Patentamt
European Patent Office
Office européen des brevets

## EUROPEAN SEARCH REPORT

Application Number

EP 23 16 2288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2022/402989 A1 (WU JIAXI [US] ET AL) 22 December 2022 (2022-12-22) * the whole document * * in particular, paragraphs 5, 13, 14, 16 and 192-193 * | 1-15 | INV. C07K16/24 C07K16/28 |
| A | CODARRI DEAK LAURA ET AL: "PD-1-cis IL-2R agonism yields better effectors from stem-like CD8+ T cells", NATURE, vol. 610, no. 7930, 28 September 2022 (2022-09-28), pages 161-172, XP093071223, London ISSN: 0028-0836, DOI: 10.1038/s41586-022-05192-0 Retrieved from the Internet: URL:https://www.nature.com/articles/s41586-022-05192-0> * the whole document * * in particular, abstract and pages 162, 164 and 169-171 * | 1-15 | |
| A | RICHTER K. ET AL: "39P ANV419 is a novel CD122-biased IL-2/anti-IL-2 fusion protein with potent CD8 T cell and NK cell stimulating capacity that shows additive efficacy in combination with checkpoint inhibitors and treatments acting through antibody dependent cellular cytotoxicity", ANNALS OF ONCOLOGY, vol. 33, 1 September 2022 (2022-09-01), page S558, XP093071324, ISSN: 0923-7534, DOI: 10.1016/j.annonc.2022.07.066 * the whole document * | 1-15 | TECHNICAL FIELDS SEARCHED (IPC) C07K A61K |

-/--

The present search report has been drawn up for all claims

| Place of search | Date of completion of the search | Examiner |
|---|---|---|
| Munich | 9 August 2023 | Pérez-Mato, Isabel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

52

## EUROPEAN SEARCH REPORT

Application Number

EP 23 16 2288

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2018/234862 A1 (MEDICENNA THERAPEUTICS INC [CA]) 27 December 2018 (2018-12-27) * the whole document * * in particular, pages 2-5 * | 1-15 | |
| Y,D | WO 2017/122130 A1 (NOVARTIS AG [CH]; UNIV ZUERICH [CH]) 20 July 2017 (2017-07-20) * the whole document * * in particular, pages 23-27, 39-42 and 85-92 * | 1-15 | |
| Y | J. TOMALA ET AL: "In Vivo Expansion of Activated Naive CD8+ T Cells and NK Cells Driven by Complexes of IL-2 and Anti-IL-2 Monoclonal Antibody As Novel Approach of Cancer Immunotherapy", THE JOURNAL OF IMMUNOLOGY, vol. 183, no. 8, 15 October 2009 (2009-10-15), pages 4904-4912, XP055369385, US ISSN: 0022-1767, DOI: 10.4049/jimmunol.0900284 * the whole document * * in particular, pages 4905, 4909 and 4910 * | 1-15 | TECHNICAL FIELDS SEARCHED (IPC) |

-/--

The present search report has been drawn up for all claims

| Place of search | Date of completion of the search | Examiner |
|---|---|---|
| Munich | 9 August 2023 | Pérez-Mato, Isabel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | S. LETOURNEAU ET AL: "IL-2/anti-IL-2 antibody complexes show strong biological activity by avoiding interaction with IL-2 receptor subunit CD25", PROCEEDINGS OF THE NATIONAL ACADEMY OF SCIENCES, vol. 107, no. 5, 19 January 2010 (2010-01-19), pages 2171-2176, XP055182715, ISSN: 0027-8424, DOI: 10.1073/pnas.0909384107 * the whole document * * in particular, page 2174 * ----- | 1-15 | |
| Y,D | WO 2013/184942 A1 (ALKERMES INC [US]) 12 December 2013 (2013-12-12) * the whole document * * in particular, pages 44-47 and SEQ ID NO:24 * ----- | 7 | |
| Y | WO 2018/184965 A1 (HOFFMANN LA ROCHE [CH]; HOFFMANN LA ROCHE [US]) 11 October 2018 (2018-10-11) * the whole document * * in particular, pages 9 and 37-39 * ----- | 10-12 | TECHNICAL FIELDS SEARCHED (IPC) |

The present search report has been drawn up for all claims

| Place of search | Date of completion of the search | Examiner |
|---|---|---|
| Munich | 9 August 2023 | Pérez-Mato, Isabel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 2288

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2023

| Patent document cited in search report | Publication date | Patent family member(s) | | Publication date |
|---|---|---|---|---|
| US 2022402989 A1 | 22-12-2022 | TW | 202317623 A | 01-05-2023 |
| | | US | 2022402989 A1 | 22-12-2022 |
| | | WO | 2022266598 A1 | 22-12-2022 |
| WO 2018234862 A1 | 27-12-2018 | AU | 2018287317 A1 | 06-02-2020 |
| | | CA | 3067909 A1 | 27-12-2018 |
| | | CN | 111201035 A | 26-05-2020 |
| | | EP | 3641814 A1 | 29-04-2020 |
| | | JP | 2020524174 A | 13-08-2020 |
| | | US | 2019062395 A1 | 28-02-2019 |
| | | US | 2023080403 A1 | 16-03-2023 |
| | | WO | 2018234862 A1 | 27-12-2018 |
| WO 2017122130 A1 | 20-07-2017 | AU | 2017208133 A1 | 05-07-2018 |
| | | BR | 112018014150 A2 | 11-12-2018 |
| | | CA | 3009001 A1 | 20-07-2017 |
| | | CN | 108473569 A | 31-08-2018 |
| | | CN | 116003593 A | 25-04-2023 |
| | | EA | 201891582 A1 | 28-12-2018 |
| | | EP | 3402819 A1 | 21-11-2018 |
| | | HK | 1257518 A1 | 25-10-2019 |
| | | IL | 260218 A | 31-07-2018 |
| | | JP | 6993699 B2 | 03-02-2022 |
| | | JP | 2019511200 A | 25-04-2019 |
| | | KR | 20180100224 A | 07-09-2018 |
| | | MA | 43859 A | 21-11-2018 |
| | | US | 2019016797 A1 | 17-01-2019 |
| | | US | 2021246200 A1 | 12-08-2021 |
| | | WO | 2017122130 A1 | 20-07-2017 |
| WO 2013184942 A1 | 12-12-2013 | AU | 2013271541 A1 | 23-10-2014 |
| | | AU | 2013271542 A1 | 16-10-2014 |
| | | AU | 2013271545 A1 | 16-10-2014 |
| | | CA | 2869674 A1 | 12-12-2013 |
| | | CA | 2869786 A1 | 12-12-2013 |
| | | CA | 2869787 A1 | 12-12-2013 |
| | | CY | 1120522 T1 | 10-07-2019 |
| | | DK | 2859015 T3 | 18-06-2018 |
| | | DK | 3401402 T3 | 11-01-2021 |
| | | EP | 2858672 A2 | 15-04-2015 |
| | | EP | 2859015 A1 | 15-04-2015 |
| | | EP | 2874639 A2 | 27-05-2015 |
| | | EP | 3401402 A1 | 14-11-2018 |
| | | EP | 3825405 A1 | 26-05-2021 |
| | | ES | 2667558 T3 | 11-05-2018 |
| | | ES | 2675568 T3 | 11-07-2018 |

EPO FORM P0459

For more details about this annex : see Official Journal of the European Patent Office, No. 12/82

page 1 of 3

## ANNEX TO THE EUROPEAN SEARCH REPORT
## ON EUROPEAN PATENT APPLICATION NO.

EP 23 16 2288

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2023

| Patent document cited in search report | Publication date | Patent family member(s) | | Publication date |
|---|---|---|---|---|
| | | ES | 2675784 T3 | 12-07-2018 |
| | | ES | 2842677 T3 | 14-07-2021 |
| | | HK | 1203820 A1 | 06-11-2015 |
| | | HK | 1203969 A1 | 06-11-2015 |
| | | HK | 1210055 A1 | 15-04-2016 |
| | | HR | P20180914 T1 | 27-07-2018 |
| | | HR | P20201973 T1 | 05-02-2021 |
| | | HU | E037849 T2 | 28-09-2018 |
| | | HU | E052908 T2 | 28-05-2021 |
| | | JP | 6234446 B2 | 22-11-2017 |
| | | JP | 6322626 B2 | 09-05-2018 |
| | | JP | 6388408 B2 | 12-09-2018 |
| | | JP | 6545759 B2 | 17-07-2019 |
| | | JP | 7048543 B2 | 05-04-2022 |
| | | JP | 2015520193 A | 16-07-2015 |
| | | JP | 2015525216 A | 03-09-2015 |
| | | JP | 2015525217 A | 03-09-2015 |
| | | JP | 2018038389 A | 15-03-2018 |
| | | JP | 2019195332 A | 14-11-2019 |
| | | JP | 2022050391 A | 30-03-2022 |
| | | LT | 2859015 T | 10-07-2018 |
| | | LT | 3401402 T | 28-12-2020 |
| | | ME | 03079 B | 20-01-2019 |
| | | NZ | 630848 A | 29-07-2016 |
| | | NZ | 630849 A | 29-04-2016 |
| | | NZ | 630851 A | 29-07-2016 |
| | | NZ | 715099 A | 21-12-2018 |
| | | PL | 2859015 T3 | 31-08-2018 |
| | | PL | 3401402 T3 | 31-05-2021 |
| | | PT | 2859015 T | 26-06-2018 |
| | | PT | 3401402 T | 11-11-2020 |
| | | SI | 2859015 T1 | 31-12-2018 |
| | | SI | 3401402 T1 | 31-03-2021 |
| | | TR | 201808753 T4 | 23-07-2018 |
| | | TR | 201809046 T4 | 23-07-2018 |
| | | US | 2013336924 A1 | 19-12-2013 |
| | | US | 2013336925 A1 | 19-12-2013 |
| | | US | 2013338067 A1 | 19-12-2013 |
| | | US | 2014234962 A1 | 21-08-2014 |
| | | US | 2016052983 A1 | 25-02-2016 |
| | | US | 2016237132 A1 | 18-08-2016 |
| | | US | 2017044228 A1 | 16-02-2017 |
| | | US | 2020040053 A1 | 06-02-2020 |
| | | WO | 2013184938 A2 | 12-12-2013 |
| | | WO | 2013184939 A2 | 12-12-2013 |
| | | WO | 2013184942 A1 | 12-12-2013 |

EPO FORM P0459

For more details about this annex : see Official Journal of the European Patent Office, No. 12/82

page 2 of 3

EP 4 431 525 A1

## ANNEX TO THE EUROPEAN SEARCH REPORT
## ON EUROPEAN PATENT APPLICATION NO.

EP 23 16 2288

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2023

| Patent document cited in search report | Publication date | Patent family member(s) | | Publication date |
|---|---|---|---|---|
| WO 2018184965 A1 | 11-10-2018 | AR | 111203 A1 | 12-06-2019 |
| | | CN | 110382525 A | 25-10-2019 |
| | | EP | 3606947 A1 | 12-02-2020 |
| | | JP | 7148539 B2 | 05-10-2022 |
| | | JP | 2020512814 A | 30-04-2020 |
| | | TW | 201842937 A | 16-12-2018 |
| | | US | 2018326011 A1 | 15-11-2018 |
| | | US | 2023071733 A1 | 09-03-2023 |
| | | WO | 2018184965 A1 | 11-10-2018 |

REFERENCES CITED IN THE DESCRIPTION

**Patent documents cited in the description**

- WO 2018184964 A1
- WO 2013184942 A1
- WO 2009080253 A
- WO 2017122130 A1
- WO 2012107417 A1
- WO 2009080253 A1
- WO 2012130831 A1

**Non-patent literature cited in the description**

- **KLEIN C.** *Oncoimmunology,* 2017, vol. 6, e1277306
- **DEAK L. C. et al.** *Nature,* 2022, vol. 610, 161
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Nat. Acad. Sci.,* 1988, vol. 85, 2444
- **DEAK LC. et al.** *Nature,* 2022, vol. 610, 161
- **GUTBRODT KL.** *Sci. Trans. Med,* 2013, vol. 5, 201
- **GILLIES SD.** *PNAS,* 1992, vol. 89 (4), 1428
- **ARENA-RAMIRES et al.** *Sci. Trans. Med.,* 2016, vol. 8, 367
- **FENWICK C.** *J Exp Med,* 2019, vol. 216 (7), 1525
- **ADLER AS.** *mAbs,* 2017
- **ROBB RJ. et al.** *J Exp. Med.,* 1987